# EUROPEAN PATENT APPLICATION

(11) **EP 3 575 396 A1**
(43) Date of publication of application: **04.12.2019**
(21) Application number: 18175634.7
(22) Date of filing: 01.06.2018
(51) Int. Cl.: C12N 9/22, C12N 15/62

(54) **GENE TARGETING**

(71) Applicant: Algentech SAS, 91058 Evry Cedex (FR)
(72) Inventor: The designation of the inventor has not yet been filed
(74) Representative: Eyre, David Edward

(57) **Abstract**

Methods, reagents and compositions for providing more accurate and reliable genetic modification are provided. In particular, a nucleic acid encoding a fusion protein comprising a 5' to 3' DNA exonuclease domain and an RNA binding domain is described. Also provided are methods, reagents and compositions for in vivo genetic modification of the genome of a human or animal cell. Furthermore, the present application relates to uses of the said methods, reagents and compositions in the treatment of disease and production of transgenic animals.

SEQUENCE LISTING

<110>  Algentech SAS

<120>  GENE TARGETING

<130>  P404655P

<160>  21

<170>  PatentIn version 3.5

<210>  1
<211>  170
<212>  DNA
<213>  Porcine circovirus

<400>  1
ctgcgggggc gggtccttct tctgcggtaa cgcctccttg gccacgtcat cctataaaag      60

tgaaagaagt gcgctgctgt agtattacca gcgcacttcg gcagcggcag cacctcggca     120

gcgtcagtga aaatgccaag caagaaaagc ggcccgcaac cccataagag                170


<210>  2
<211>  939
<212>  DNA
<213>  Porcine circovirus

<400>  2
atgccaagca agaaaagcgg cccgcaaccc cataagaggt gggtgttcac ccttaataat      60

ccttccgagg aggagaaaaa caaaatacgg gagcttccaa tctccctttt tgattatttt     120

gtttgcggag aggaaggttt ggaagagggt agaactcctc acctccaggg gtttgcgaat     180

tttgctaaga agcagacttt taacaaggtg aagtggtatt ttggtgcccg ctgccacatc     240

gagaaagcga aaggaaccga ccagcagaat aaagaatact gcagtaaaga aggtcacata     300

cttatcgagt gtggagctcc gcggaaccag gggaagcgca gcgacctgtc tactgctgtg     360

agtacccttt tggagacggg gtctttggtg actgtagccg agcagttccc tgtaacgtat     420

gtgagaaatt tccgcgggct ggctgaactt ttgaaagtga gcgggaagat gcagcagcgt     480

gattggaaga cagctgtaca cgtcatagtg ggcccgcccg gttgtgggaa gagccagtgg     540

gcccgtaatt ttactgagcc tagcgacacc tactggaagc ctagtagaaa taagtggtgg     600

gatggatatc atggagaaga agttgttgtt ttggatgatt tttatggctg gttaccttgg     660

gatgatctac tgagactgtg tgaccggtat ccattgactg tagagactaa aggcggtact     720

gttccttttt tggcccgcag tattttgatt accagcaatc aggcccccca ggaatggtac     780

tcctcaactg ctgtcccagc tgtagaagct ctctatcgga ggattactac tttgcaattt     840

tggaagactg ctggagaaca atccacggag gtacccgaag gccgatttga agcagtggac     900

ccaccctgtg cccttttccc atataaaata aattactga                           939

<210>  3
<211>  1501
<212>  DNA
<213>  Artificial Sequence

<220>
<223>  Topoisomerase I donor gene - camptothecin sensitive mutant

<400>  3
gtccggcaga gagctgtagc cctgtacttc atcgacaagc ttgctctgag agcaggcaat      60

gaaaaggagg aaggagaaac agcggacact gtgggctgct gctcacttcg tgtggagcac     120

atcaatctac acccagagtt ggatggtcag gaatatgtgg tagagtttga cttcctcggg     180

aaggactcca tcagatacta taacaaggtc cctgttgaga aacgagtttt taagaaccta     240

caactattta tggagaacaa gcagcccgag gatgatcttt ttgatagact caatactggt     300

attctgaata agcatcttca ggatctcatg gagggcttga cagccaaggt attccgtacg     360

tacaatgcct ccatcacgct acagcagcag ctaaaagaac tgacagcccc ggatgagaac     420

atcccagcga agatcctttc ttataaccgt gccaatcgag ctgttgcaat tctttgtaac     480

catcagaggg caccaccaaa aacttttgag aagtctatga tgaacttgca aactaagatt     540

gatgccaaga aggaacagct agcagatgcc cggagagacc tgaaaagtgc taaggctgat     600

gccaaggtca tgaaggatgc aaagacgaag aaggtagtag agtcaaagaa gaaggctgtt     660

cagagactgg aggaacagtt gatgaagctg gaagttcaag ccacagaccg agaggaaaat     720

aaacagattg ccctgggaac ctccaaactc agctatctgg accctaggat cacagtggct     780

tggtgcaaga agtggggtgt cccaattgag aagatttaca acaaaaccca gcgggagaag     840

tttgcctggg ccattgacat ggctgatgaa gactatgagt tttagccagt ctcaagaggc     900

agagttctgt gaagaggaac agtgtggttt gggaaagatg gataaactga gcctcacttg     960

ccctcgtgcc tgggggagag aggcagcaag tcttaacaaa ccaacatctt tgcgaaaaga    1020

taaacctgga gatattataa gggagagctg agccagttgt cctatggaca acttatttaa    1080

aaatatttca gatatcaaaa ttctagctgt atgatttgtt ttgaattttg tttttatttt    1140

caagagggca agtggatggg aatttgtcag cgttctacca ggcaaattca ctgtttcact    1200

gaaatgtttg gattctctta gctactgtat gcaaagtccg attatattgg tgcgttttta    1260

cagttagggt tttgcaataa cttctatatt ttaatagaaa taaattccta aactcccttc    1320

cctctctccc atttcaggaa tttaaaatta agtagaacaa aaaacccagc gcacctgtta    1380

gagtcgtcac tctctattgt catggggatc aattttcatt aaacttgaag cagtcgtggc    1440

tttggcagtg ttttggttca gacacctgtt cacagaaaaa gcatgatggg aaaatatttc    1500

c                                                                    1501

<210>  4
<211>  2311
<212>  DNA
<213>  Artificial Sequence

<220>
<223>  Topoisomerase I wtih eGFP insertion donor gene

<400>  4
cagcggacac tgtgggctgc tgctcacttc gtgtggagca catcaatcta cacccagagt      60

tggatggtca ggaatatgtg gtagagtttg acttcctcgg gaaggactcc atcagatact     120

ataacaaggt ccctgttgag aaacgagttt ttaagaacct acaactattt atggagaaca     180

agcagcccga ggatgatctt tttgatagac tcaatactgg tattctgaat aagcatcttc     240

aggatctcat ggagggcttg acagccaagg tattccgtac gtacaatgcc tccatcacgc     300

tacagcagca gctaaaagaa ctgacagccc cggatgagaa catcccagcg aagatccttt     360

cttataaccg tgccaatcga gctgttgcaa ttctttgtaa ccatcagagg gcaccaccaa     420

aaacttttga gaagtctatg atgaacttgc aaactaagat tgatgccaag aaggaacagc     480

tagcagatgc ccggagagac ctgaaaagtg ctaaggctga tgccaaggtc atgaaggatg     540

caaagacgaa gaaggtagta gagtcaaaga agaaggctgt tcagagactg gaggaacagt     600

tgatgaagct ggaagttcaa gccacagacc gagaggaaaa taaacagatt gccctgggaa     660

cctccaaact cagctatctg gaccctagga tcacagtggc ttggtgcaag aagtggggtg     720

tcccaattga gaagatttac aacaaaaccc agcgggagaa gtttgcctgg gccattgaca     780

tggctgatga agactatgag tttaagggcg aggagctgtt caccggggtg gtgcccatcc     840

tggtcgagct ggacggcgac gtaaacggcc acaagttcag cgtgtccggc gagggcgagg     900

gcgatgccac ctacggcaag ctgaccctga agttcatctg caccaccggc aagctgcccg     960

tgccctggcc caccctcgtg accaccctga cctacggcgt gcagtgcttc agccgctacc    1020

ccgaccacat gaagcagcac gacttcttca agtccgccat gcccgaaggc tacgtccagg    1080

agcgcaccat cttcttcaag gacgacggca actacaagac ccgcgccgag gtgaagttcg    1140

agggcgacac cctggtgaac cgcatcgagc tgaagggcat cgacttcaag gaggacggca    1200

acatcctggg gcacaagctg gagtacaact acaacagcca caacgtctat atcatggccg    1260

acaagcagaa gaacggcatc aaggtgaact tcaagatccg ccacaacatc gaggacggca    1320

gcgtgcagct cgccgaccac taccagcaga acacccccat cggcgacggc cccgtgctgc    1380

tgcccgacaa ccactacctg agcacccagt ccgccctgag caaagacccc aacgagaagc    1440

gcgatcacat ggtcctgctg gagttcgtga ccgccgccgg gatcactctc ggcatggacg    1500

agctatacaa gccagtctca agaggcagag ttctgtgaag aggaacagtg tggtttggga    1560

aagatggata aactgagcct cacttgccct cgtgcctggg ggagagaggc agcaagtctt    1620

aacaaaccaa catctttgcg aaaagataaa cctggagata ttataaggga gagctgagcc 1680

agttgtccta tggacaactt atttaaaaat atttcagata tcaaaattct agctgtatga 1740

tttgttttga attttgtttt tattttcaag agggcaagtg gatgggaatt tgtcagcgtt 1800

ctaccaggca aattcactgt ttcactgaaa tgtttggatt ctcttagcta ctgtatgcaa 1860

agtccgatta tattggtgcg tttttacagt tagggttttg caataacttc tatattttaa 1920

tagaaataaa ttcctaaact cccttccctc tctcccattt caggaattta aaattaagta 1980

gaacaaaaaa cccagcgcac ctgttagagt cgtcactctc tattgtcatg gggatcaatt 2040

ttcattaaac ttgaagcagt cgtggctttg gcagtgtttt ggttcagaca cctgttcaca 2100

gaaaaagcat gatgggaaaa tatttcctga cttgagtgtt cctttttaaa tgtgaatttt 2160

tttttttttt aattatttta aaatatttaa acctttttct tgatcttaaa gatcgtgtag 2220

attggggttg gggagggatg aagggcgagt gaatctaagg ataatgaaat aatcagtgac 2280

tgaaaccatt ttcccatcat cctttgttct g 2311

<210> 5
<211> 1331
<212> DNA
<213> Human cytomegalovirus

<400> 5
caatcccaca aaaatctgag cttaacagca cagttgctcc tctcagagca gaatcgggta 60

ttcaacaccc tcatatcaac tactacgttg tgtataacgg tccacatgcc ggtatatacg 120

atgactgggg ttgtacaaag gcggcaacaa acggcgttcc cggagttgca cacaagaaat 180

ttgccactat tacagaggca agagcagcag ctgacgcgta cacaacaagt cagcaaacag 240

acaggttgaa cttcatcccc aaaggagaag ctcaactcaa gcccaagagc tttgctaagg 300

ccctaacaag cccaccaaag caaaaagccc actggctcac gctaggaacc aaaaggccca 360

gcagtgatcc agccccaaaa gagatctcct ttgccccgga gattacaatg gacgatttcc 420

tctatcttta cgatctagga aggaagttcg aaggtgaagt agacgacact atgttcacca 480

ctgataatga gaaggttagc ctcttcaatt tcagaaagaa tgctgaccca cagatggtta 540

gagaggccta cgcagcaggt ctcatcaaga cgatctaccc gagtaacaat ctccaggaga 600

tcaaatacct tcccaagaag gttaaagatg cagtcaaaag attcaggact aattgcatca 660

agaacacaga gaaagacata tttctcaaga tcagaagtac tattccagta tggacgattc 720

aaggcttgct tcataaacca aggcaagtaa tagagattgg agtctctaaa aaggtagttc 780

ctactgaatc taaggccatg catggagtct aagattcaaa tcgaggatct aacagaactc 840

gccgtgaaga ctggcgaaca gttcatacag agtcttttac gactcaatga caagaagaaa 900

atcttcgtca acatggtgga gcacgacact ctggtctact ccaaaaatgt caaagataca 960

gtctcagaag accaaagggc tattgagact tttcaacaaa ggataatttc gggaaacctc 1020

ctcggattcc attgcccagc tatctgtcac ttcatcgaaa ggacagtaga aaaggaaggt 1080

ggctcctaca aatgccatca ttgcgataaa ggaaaggcta tcattcaaga tctctctgcc 1140

gacagtggtc ccaaagatgg acccccaccc acgaggagca tcgtggaaaa agaagacgtt 1200

ccaaccacgt cttcaaagca agtggattga tgtgacatct ccactgacgt aagggatgac 1260

gcacaatccc actatccttc gcaagaccct tcctctatat aaggaagttc atttcatttg 1320

gagaggacac g 1331

<210> 6
<211> 4140
<212> DNA
<213> Streptococcus pyogenes

<400> 6
atggacaaga agtacagcat cggcctggac atcggcacga actcggtggg ctgggcggtg 60

atcacggacg agtacaaggt gccctccaag aagttcaagg tgctgggcaa caccgaccgc 120

cactcgatca agaagaacct gatcggcgcc ctgctgttcg actccggcga gaccgccgag 180

gcgacgcgcc tgaagcgcac cgcgcgtcgc cgctacacgc gtcgcaagaa ccgcatctgc 240

tacctgcagg agatcttcag caacgagatg gccaaggtgg acgactcgtt cttccaccgc 300

ctggaggagt ccttcctggt ggaggaagac aagaagcacg agcgccaccc catcttcggc 360

aacatcgtgg acgaggtggc ctaccacgag aagtacccga cgatctacca cctgcgcaag 420

aagctggtgg acagcaccga caaggcggac ctgcgcctga tctacctggc cctggcgcac 480

atgatcaagt tccgcggcca cttcctgatc gagggcgacc tgaaccccga caactcggac 540

gtggacaagc tgttcatcca gctggtgcag acctacaacc agctgttcga ggagaacccg 600

atcaacgcct ccggcgtgga cgccaaggcg atcctgagcg cgcgcctgtc caagagccgt 660

cgcctggaga acctgatcgc ccagctgccc ggcgagaaga agaacggcct gttcggcaac 720

ctgatcgcgc tgtcgctggg cctgacgccg aacttcaagt ccaacttcga cctggccgag 780

gacgcgaagc tgcagctgag caaggacacc tacgacgacg acctggacaa cctgctggcc 840

cagatcggcg accagtacgc ggacctgttc ctggccgcga agaacctgtc ggacgccatc 900

ctgctgtccg acatcctgcg cgtgaacacc gagatcacga aggcccccct gtcggcgtcc 960

atgatcaagc gctacgacga gcaccaccag gacctgaccc tgctgaaggc gctggtgcgc 1020

cagcagctgc cggagaagta caaggagatc ttcttcgacc agagcaagaa cggctacgcc 1080

ggctacatcg acggcggcgc gtcgcaagag gagttctaca agttcatcaa gcccatcctg 1140

gagaagatgg acggcacgga ggagctgctg gtgaagctga accgcgagga cctgctgcgc 1200

aagcagcgca ccttcgacaa cggcagcatc ccccaccaga tccacctggg cgagctgcac 1260

gccatcctgc gtcgccaaga ggacttctac ccgttcctga aggacaaccg cgagaagatc 1320

gagaagatcc tgacgttccg catcccctac tacgtgggcc cgctggcccg cggcaacagc 1380

cgcttcgcgt ggatgacccg caagtcggag gagaccatca cgccctggaa cttcgaggaa 1440

gtggtggaca agggcgccag cgcgcagtcg ttcatcgagc gcatgaccaa cttcgacaag 1500

aacctgccca acgagaaggt gctgccgaag cactccctgc tgtacgagta cttcaccgtg 1560

tacaacgagc tgacgaaggt gaagtacgtg accgagggca tgcgcaagcc cgccttcctg 1620

agcggcgagc agaagaaggc gatcgtggac ctgctgttca agaccaaccg caaggtgacg 1680

gtgaagcagc tgaaagagga ctacttcaag aagatcgagt gcttcgacag cgtggagatc 1740

tcgggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga cgctgttcga ggaccgcgag atgatcgagg agcgcctgaa gacgtacgcc 1920

cacctgttcg acgacaaggt gatgaagcag ctgaagcgtc gccgctacac cggctggggc 1980

cgcctgagcc gcaagctgat caacggcatc cgcgacaagc agtccggcaa gaccatcctg 2040

gacttcctga agagcgacgg cttcgcgaac cgcaacttca tgcagctgat ccacgacgac 2100

tcgctgacct tcaaagagga catccagaag gcccaggtgt cgggccaggg cgactccctg 2160

cacgagcaca tcgccaacct ggcgggctcc cccgcgatca agaagggcat cctgcagacc 2220

gtgaaggtgg tggacgagct ggtgaaggtg atgggccgcc acaagccgga gaacatcgtg 2280

atcgagatgg cccgcgagaa ccagaccacg cagaagggcc agaagaacag ccgcgagcgc 2340

atgaagcgca tcgaggaagg catcaaggag ctgggctcgc agatcctgaa ggagcacccc 2400

gtggagaaca cccagctgca gaacgagaag ctgtacctgt actacctgca gaacggccgc 2460

gacatgtacg tggaccagga gctggacatc aaccgcctgt ccgactacga cgtggaccac 2520

atcgtgcccc agagcttcct gaaggacgac tcgatcgaca acaaggtgct gacccgcagc 2580

gacaagaacc gcggcaagag cgacaacgtg ccgtcggagg aagtggtgaa gaagatgaag 2640

aactactggc gccagctgct gaacgccaag ctgatcacgc agcgcaagtt cgacaacctg 2700

accaaggccg agcgcggtgg cctgtcggag ctggacaagg cgggcttcat caagcgccag 2760

ctggtggaga cccgccagat cacgaagcac gtggcgcaga tcctggactc ccgcatgaac 2820

acgaagtacg acgagaacga caagctgatc cgcgaggtga aggtgatcac cctgaagtcc 2880

aagctggtca gcgacttccg caaggacttc cagttctaca aggtgcgcga gatcaacaac 2940

taccaccacg cccacgacgc gtacctgaac gccgtggtgg gcaccgcgct gatcaagaag 3000

taccccaagc tggagagcga gttcgtgtac ggcgactaca aggtgtacga cgtgcgcaag 3060

atgatcgcca agtcggagca ggagatcggc aaggccaccg cgaagtactt cttctactcc 3120

aacatcatga acttcttcaa gaccgagatc acgctggcca acggcgagat ccgcaagcgc 3180

ccgctgatcg agaccaacgg cgagacgggc gagatcgtgt gggacaaggg ccgcgacttc 3240

gcgaccgtgc gcaaggtgct gagcatgccc caggtgaaca tcgtgaagaa gaccgaggtg 3300

cagacgggcg gcttctccaa ggagagcatc ctgccgaagc gcaactcgga caagctgatc 3360

gcccgcaaga aggactggga ccccaagaag tacggcggct tcgactcccc gaccgtggcc 3420

tacagcgtgc tggtggtggc gaaggtggag aagggcaagt ccaagaagct gaagagcgtg 3480

aaggagctgc tgggcatcac catcatggag cgcagctcgt tcgagaagaa ccccatcgac 3540

ttcctggagg ccaagggcta caaagaggtg aagaaggacc tgatcatcaa gctgccgaag 3600

tactcgctgt tcgagctgga gaacggccgc aagcgcatgc tggcctccgc gggcgagctg 3660

cagaagggca acgagctggc cctgcccagc aagtacgtga acttcctgta cctggcgtcc 3720

cactacgaga agctgaaggg ctcgccggag gacaacgagc agaagcagct gttcgtggag 3780

cagcacaagc actacctgga cgagatcatc gagcagatct cggagttctc caagcgcgtg 3840

atcctggccg acgcgaacct ggacaaggtg ctgagcgcct acaacaagca ccgcgacaag 3900

cccatccgcg agcaggcgga gaacatcatc cacctgttca ccctgacgaa cctgggcgcc 3960

ccggccgcgt tcaagtactt cgacaccacg atcgaccgca agcgctacac ctccacgaaa 4020

gaggtgctgg acgcgaccct gatccaccag agcatcaccg gcctgtacga gacgcgcatc 4080

gacctgagcc agctgggcgg cgactcccgc gcggacccga agaagaagcg caaggtgtaa 4140

<210> 7
<211> 232
<212> DNA
<213> Homo sapiens

<400> 7
tttcccatga ttccttcata tttgcatata cgatacaagg ctgttagaga gataattgga 60

attaatttga ctgtaaacac aaagatatta gtacaaaata cgtgacgtag aaagtaataa 120

tttcttgggt agtttgcagt tttaaaatta tgttttaaaa tggactatca tatgcttacc 180

gtaacttgaa agtatttcga tttcttggct ttatatatct tgtggaaagg ac 232

<210> 8
<211> 167
<212> DNA
<213> Artificial Sequence

<220>
<223> sgRNA2.0 with MS2 hairpins for modification of topoisomerase I
 gene

<400> 8
gatttacaac aaaacccagc gttttagagc taggccaaca tgaggatcac ccatgtctgc 60

agggcctagc aagttaaaat aaggctagtc cgttatcaac ttggccaaca tgaggatcac 120

ccatgtctgc agggccaagt ggcaccgagt cggtgctttt ttttttt 167

<210> 9
<211> 222
<212> DNA
<213> Simian virus 40

<400> 9
cagacatgat aagatacatt gatgagtttg gacaaaccac aactagaatg cagtgaaaaa 60

aatgctttat ttgtgaaatt tgtgatgcta ttgctttatt tgtaaccatt ataagctgca 120

ataaacaagt taacaacaac aattgcattc attttatgtt tcaggttcag ggggaggtgt 180

gggaggtttt ttaaagcaag taaaacctct acaaatgtgg ta 222

<210> 10
<211> 5271
<212> DNA
<213> Artificial Sequence

<220>
<223> cas9-B-PCV1 rep fusion protein gene

<400> 10
atggactata aggaccacga cggagactac aaggatcatg atattgatta caaagacgat 60

gacgataaga tggccccaaa gaagaagcgg aaggtcggta tccacggagt cccagcagcc 120

gacaagaagt acagcatcgg cctggacatc ggcaccaact ctgtgggctg ggccgtgatc 180

accgacgagt acaaggtgcc cagcaagaaa ttcaaggtgc tgggcaacac cgaccggcac 240

agcatcaaga agaacctgat cggagccctg ctgttcgaca gcggcgaaac agccgaggcc 300

acccggctga agagaaccgc cagaagaaga tacaccagac ggaagaaccg gatctgctat 360

ctgcaagaga tcttcagcaa cgagatggcc aaggtggacg acagcttctt ccacagactg 420

gaagagtcct tcctggtgga agaggataag aagcacgagc ggcaccccat cttcggcaac 480

atcgtggacg aggtggccta ccacgagaag taccccacca tctaccacct gagaaagaaa 540

ctggtggaca gcaccgacaa ggccgacctg cggctgatct atctggccct ggcccacatg 600

atcaagttcc ggggccactt cctgatcgag ggcgacctga accccgacaa cagcgacgtg 660

gacaagctgt tcatccagct ggtgcagacc tacaaccagc tgttcgagga aaaccccatc 720

aacgccagcg gcgtggacgc caaggccatc ctgtctgcca gactgagcaa gagcagacgg 780

ctggaaaatc tgatcgccca gctgcccggc gagaagaaga atggcctgtt cggaaacctg 840

attgccctga gcctgggcct gacccccaac ttcaagagca acttcgacct ggccgaggat 900

gccaaactgc agctgagcaa ggacacctac gacgacgacc tggacaacct gctggcccag 960

atcggcgacc agtacgccga cctgtttctg gccgccaaga acctgtccga cgccatcctg 1020

ctgagcgaca tcctgagagt gaacaccgag atcaccaagg cccccctgag cgcctctatg 1080

atcaagagat acgacgagca ccaccaggac ctgaccctgc tgaaagctct cgtgcggcag 1140

cagctgcctg agaagtacaa agagattttc ttcgaccaga gcaagaacgg ctacgccggc 1200

tacattgacg gcggagccag ccaggaagag ttctacaagt tcatcaagcc catcctggaa 1260

aagatggacg gcaccgagga actgctcgtg aagctgaaca gagaggacct gctgcggaag 1320

cagcggacct tcgacaacgg cagcatcccc caccagatcc acctgggaga gctgcacgcc 1380

attctgcggc ggcaggaaga tttttaccca ttcctgaagg acaaccggga aaagatcgag 1440

aagatcctga ccttccgcat cccctactac gtgggccctc tggccagggg aaacagcaga 1500

ttcgcctgga tgaccagaaa gagcgaggaa accatcaccc cctggaactt cgaggaagtg 1560

gtggacaagg gcgcttccgc ccagagcttc atcgagcgga tgaccaactt cgataagaac 1620

ctgcccaacg agaaggtgct gcccaagcac agcctgctgt acgagtactt caccgtgtat 1680

aacgagctga ccaaagtgaa atacgtgacc gagggaatga gaaagcccgc cttcctgagc 1740

ggcgagcaga aaaaggccat cgtggacctg ctgttcaaga ccaaccggaa agtgaccgtg 1800

aagcagctga aagaggacta cttcaagaaa atcgagtgct tcgactccgt ggaaatctcc 1860

ggcgtggaag atcggttcaa cgcctccctg ggcacatacc acgatctgct gaaaattatc 1920

aaggacaagg acttcctgga caatgaggaa aacgaggaca ttctggaaga tatcgtgctg 1980

accctgacac tgtttgagga cagagagatg atcgaggaac ggctgaaaac ctatgcccac 2040

ctgttcgacg acaaagtgat gaagcagctg aagcggcgga gatacaccgg ctggggcagg 2100

ctgagccgga agctgatcaa cggcatccgg gacaagcagt ccggcaagac aatcctggat 2160

ttcctgaagt ccgacggctt cgccaacaga aacttcatgc agctgatcca cgacgacagc 2220

ctgaccttta aagaggacat ccagaaagcc caggtgtccg gccagggcga tagcctgcac 2280

gagcacattg ccaatctggc cggcagcccc gccattaaga agggcatcct gcagacagtg 2340

aaggtggtgg acgagctcgt gaaagtgatg ggccggcaca agcccgagaa catcgtgatc 2400

gaaatggcca gagagaacca gaccacccag aagggacaga agaacagccg cgagagaatg 2460

aagcggatcg aagagggcat caaagagctg ggcagccaga tcctgaaaga acaccccgtg 2520

gaaaacaccc agctgcagaa cgagaagctg tacctgtact acctgcagaa tgggcgggat 2580

atgtacgtgg accaggaact ggacatcaac cggctgtccg actacgatgt ggaccatatc 2640

gtgcctcaga gctttctgaa ggacgactcc atcgacaaca aggtgctgac cagaagcgac 2700

aagaaccggg gcaagagcga caacgtgccc tccgaagagg tcgtgaagaa gatgaagaac 2760

tactggcggc agctgctgaa cgccaagctg attacccaga gaaagttcga caatctgacc 2820

aaggccgaga gaggcggcct gagcgaactg gataaggccg gcttcatcaa gagacagctg 2880

gtggaaaccc ggcagatcac aaagcacgtg gcacagatcc tggactcccg gatgaacact 2940

aagtacgacg agaatgacaa gctgatccgg gaagtgaaag tgatcaccct gaagtccaag 3000

ctggtgtccg atttccggaa ggatttccag ttttacaaag tgcgcgagat caacaactac 3060

caccacgccc acgacgccta cctgaacgcc gtcgtgggaa ccgccctgat caaaaagtac 3120

cctaagctgg aaagcgagtt cgtgtacggc gactacaagg tgtacgacgt gcggaagatg 3180

atcgccaaga gcgagcagga aatcggcaag gctaccgcca agtacttctt ctacagcaac 3240

atcatgaact ttttcaagac cgagattacc ctggccaacg gcgagatccg gaagcggcct 3300

ctgatcgaga caaacggcga aaccggggag atcgtgtggg ataagggccg ggattttgcc 3360

accgtgcgga aagtgctgag catgccccaa gtgaatatcg tgaaaaagac cgaggtgcag 3420

acaggcggct tcagcaaaga gtctatcctg cccaagagga acagcgataa gctgatcgcc 3480

agaaagaagg actgggaccc taagaagtac ggcggcttcg acagccccac cgtggcctat 3540

tctgtgctgg tggtggccaa agtggaaaag ggcaagtcca agaaactgaa gagtgtgaaa 3600

gagctgctgg ggatcaccat catggaaaga agcagcttcg agaagaatcc catcgacttt 3660

ctggaagcca agggctacaa agaagtgaaa aaggacctga tcatcaagct gcctaagtac 3720

tccctgttcg agctggaaaa cggccggaag agaatgctgg cctctgccgg cgaactgcag 3780

aagggaaacg aactggccct gccctccaaa tatgtgaact tcctgtacct ggccagccac 3840

tatgagaagc tgaagggctc ccccgaggat aatgagcaga aacagctgtt tgtggaacag 3900

cacaagcact acctggacga gatcatcgag cagatcagcg agttctccaa gagagtgatc 3960

ctggccgacg ctaatctgga caaagtgctg tccgcctaca acaagcaccg ggataagccc 4020

atcagagagc aggccgagaa tatcatccac ctgtttaccc tgaccaatct gggagcccct 4080

gccgccttca agtactttga caccaccatc gaccggaaga ggtacaccag caccaaagag 4140

gtgctggacg ccaccctgat ccaccagagc atcaccggcc tgtacgagac acggatcgac 4200

ctgtctcagc tgggaggcga caaaaggccg gcggccacga aaaaggccgg ccaggcaaaa 4260

aagaaaaagg tgagcgctgg aggaggtgga agcggaggag gaggaagcgg aggaggaggt 4320

agcctcgaga ccatgccaag caagaaaagc ggcccgcaac cccataagag gtgggtgttc 4380

acccttaata atccttccga ggaggagaaa aacaaaatac gggagcttcc aatctccctt 4440

tttgattatt ttgtttgcgg agaggaaggt ttggaagagg gtagaactcc tcacctccag 4500

gggtttgcga attttgctaa gaagcagact tttaacaagg tgaagtggta ttttggtgcc 4560

cgctgccaca tcgagaaagc gaaaggaacc gaccagcaga ataaagaata ttgcagtaaa 4620

gaaggtcaca tacttatcga gtgtggagcc ccccggaacc aggggaagcg cagcgacctg 4680

tctactgctg tgagtaccct tttggagacg gggtctttgg tgactgtagc cgagcagttc 4740

cctgtaacgt atgtgagaaa tttccggggg ctggctgaac ttttgaaagt gagcgggaag 4800

atgcagcagc gtgattggaa gacagctgta cacgtcatag tgggaccgcc cggttgtggg 4860

aagagccagt gggcccgtaa ttttactgag cctagcgaca cctactggaa gcctagtaga 4920

aataagtggt gggatggcta tcatggagaa gaagttgttg ttttggatga tttttatggc 4980

tggttacctt gggatgatct actgagactg tgtgaccggt atccattgac tgtagagact 5040

aaaggcggta ctgttccttt tttggcccgc agtattttga ttaccagcaa tcaggccccc 5100

caggaatggt actcctcaac tgctgtccca gccgtagaag ctctctatcg gaggattact 5160

actttgcaat tttggaagac tgctggagaa caatccacgg aggtgcccga aggccgattt 5220

gaagcagtgg acccaccctg tgcccttttc ccatataaaa taaattactg a 5271

<210> 11
<211> 681
<212> DNA
<213> Bacteriophage lambda

<400> 11
atgacaccgg acattatcct gcagcgtacc gggatcgatg tgagagctgt cgaacagggg 60

gatgatgcgt ggcacaaatt acggctcggc gtcatcaccg cttcagaagt tcacaacgtg 120

atagcaaaac cccgctccgg aaagaagtgg cctgacatga aaatgtccta cttccacacc 180

ctgcttgctg aggtttgcac cggtgtggct ccggaagtta acgctaaagc actggcctgg 240

ggaaaacagt acgagaacga cgccagaacc ctgtttgaat tcacttccgg cgtgaatgtt 300

actgaatccc cgatcatcta tcgcgacgaa agtatgcgta ccgcctgctc tcccgatggt 360

ttatgcagtg acggcaacgg ccttgaactg aaatgcccgt ttacctcccg ggatttcatg 420

aagttccggc tcggtggttt cgaggccata aagtcagctt acatggccca ggtgcagtac 480

agcatgtggg tgacgcgaaa aaatgcctgg tactttgcca actatgaccc gcgtatgaag 540

cgtgaaggcc tgcattatgt cgtgattgag cgggatgaaa agtacatggc gagttttgac 600

gagatcgtgc cggagttcat cgaaaaaatg gacgaggcac tggctgaaat tggttttgta 660

tttggggagc aatggcgatg a 681

<210> 12
<211> 155
<212> DNA
<213> Artificial Sequence

<220>
<223> sgRNA comprising multiple stem-loops

<400> 12
gttttagagc taggccaaca tgaggatcac ccatgtctgc agggcctagc aagttaaaat 60

aaggctagtc cgttatcaac tttaaggagt ttatatggaa acccttaaag tggcaccgag 120

tcggtgctac cgccgacaac gcggtttttt ttttt 155

<210> 13

<211>  3237
<212>  DNA
<213>  Artificial Sequence

<220>
<223>  Triple fusion of E.coli recA gene

<400>  13
atggacgaga acaagaagcg cgccctggcc gcggccctgg gacagatcga acgccaattc      60

ggcaaaggcg cggtcatgcg catgggcgac catgagcgcc aggcgatccc ggccatctcc     120

accggctccc tgggtctgga catcgccctc ggcatcggcg gcctgcccaa gggccggatc     180

gtcgagatct acggtccgga atcctcgggc aagaccaccc tgaccctctc ggtgatcgcc     240

gaggcccaga aacagggcgc cacctgtgcc ttcgtcgacg ccgagcacgc gctcgatccc     300

gactatgccg gcaagctggg cgtcaacgtc gacgacctgc tggtctccca gccggacacc     360

ggcgagcagg ccctggaaat caccgacatg ctggtgcgct ccaacgcggt cgacgtgatc     420

atcgtcgact ccgtggccgc gctggtaccc aaggccgaga tcgaaggcga gatgggcgac     480

gcccacgtcg gcctgcaggc acgcctgatg tcccaggcgc tgcgcaagat caccggcaat     540

atcaagaacg ccaactgcct ggtcatcttc atcaaccaga tccgcatgaa gatcggcgtc     600

atgttcggca acccggaaac caccaccggc ggtaacgcac tgaagttcta cgcctcggtc     660

cgcctggaca tccgtcgtac cggcgcggtg aaggaaggcg acgaggtggt gggtagcgaa     720

acccgcgtca aggtggtgaa gaacaaggtt tccccgccgt tccgccaggc cgagttccag     780

atcctctacg gtaagggcat ctaccgtacc ggcgagatca tcgatctggg cgtgcaattg     840

ggcctggtcg agaagtccgg cgcctggtac agctaccagg gcagcaagat cggccagggc     900

aaggcgaacg ccgccaagta cctggaagac aatccggaaa tcggttcggt actggagaag     960

accattcgcg accagttgct ggccaagagc ggcccggtga aggccgacgc cgaagaagtg    1020

gctgacgccg aagccgattc agagctcgga gaaggtcaag gacagggaca aggtccagga    1080

cgaggatacg catataagct tgacgagaac aagaagcgcg ccctggccgc ggccctggga    1140

cagatcgaac gccaattcgg caaaggcgcg gtcatgcgca tgggcgacca tgagcgccag    1200

gcgatcccgg ccatctccac cggctccctg ggtctggaca tcgccctcgg catcggcggc    1260

ctgcccaagg gccggatcgt cgagatctac ggtccggaat cctcgggcaa gaccaccctg    1320

accctctcgg tgatcgccga ggcccagaaa cagggcgcca cctgtgcctt cgtcgacgcc    1380

gagcacgcgc tcgatcccga ctatgccggc aagctgggcg tcaacgtcga cgacctgctg    1440

gtctcccagc cggacaccgg cgagcaggcc ctggaaatca ccgacatgct ggtgcgctcc    1500

aacgcggtcg acgtgatcat cgtcgactcc gtggccgcgc tggtacccaa ggccgagatc    1560

gaaggcgaga tgggcgacgc ccacgtcggc ctgcaggcac gcctgatgtc ccaggcgctg    1620

cgcaagatca ccggcaatat caagaacgcc aactgcctgg tcatcttcat caaccagatc    1680

cgcatgaaga tcggcgtcat gttcggcaac ccggaaacca ccaccggcgg taacgcactg 1740

aagttctacg cctcggtccg cctggacatc cgtcgtaccg gcgcggtgaa ggaaggcgac 1800

gaggtggtgg gtagcgaaac ccgcgtcaag gtggtgaaga acaaggtttc cccgccgttc 1860

cgccaggccg agttccagat cctctacggt aagggcatct accgtaccgg cgagatcatc 1920

gatctgggcg tgcaattggg cctggtcgag aagtccggcg cctggtacag ctaccagggc 1980

agcaagatcg gccagggcaa ggcgaacgcc gccaagtacc tggaagacaa tccggaaatc 2040

ggttcggtac tggagaagac cattcgcgac cagttgctgg ccaagagcgg cccggtgaag 2100

gccgacgccg aagaagtggc tgacgccgaa gccgattcag gatccggaga aggtcaagga 2160

cagggacaag gtccaggacg aggatacgca tatgcatgcg acgagaacaa gaagcgcgcc 2220

ctggccgcgg ccctgggaca gatcgaacgc caattcggca aaggcgcggt catgcgcatg 2280

ggcgaccatg agcgccaggc gatcccggcc atctccaccg gctccctggg tctggacatc 2340

gccctcggca tcggcggcct gcccaagggc cggatcgtcg agatctacgg tccggaatcc 2400

tcgggcaaga ccaccctgac cctctcggtg atcgccgagg cccagaaaca gggcgccacc 2460

tgtgccttcg tcgacgccga gcacgcgctc gatcccgact atgccggcaa gctgggcgtc 2520

aacgtcgacg acctgctggt ctcccagccg gacaccggcg agcaggccct ggaaatcacc 2580

gacatgctgg tgcgctccaa cgcggtcgac gtgatcatcg tcgactccgt ggccgcgctg 2640

gtacccaagg ccgagatcga aggcgagatg ggcgacgccc acgtcggcct gcaggcacgc 2700

ctgatgtccc aggcgctgcg caagatcacc ggcaatatca agaacgccaa ctgcctggtc 2760

atcttcatca accagatccg catgaagatc ggcgtcatgt tcggcaaccc ggaaaccacc 2820

accggcggta acgcactgaa gttctacgcc tcggtccgcc tggacatccg tcgtaccggc 2880

gcggtgaagg aaggcgacga ggtggtgggt agcgaaaccc gcgtcaaggt ggtgaagaac 2940

aaggtttccc cgccgttccg ccaggccgag ttccagatcc tctacggtaa gggcatctac 3000

cgtaccggcg agatcatcga tctgggcgtg caattgggcc tggtcgagaa gtccggcgcc 3060

tggtacagct accagggcag caagatcggc cagggcaagg cgaacgccgc caagtacctg 3120

gaagacaatc cggaaatcgg ttcggtactg gagaagacca ttcgcgacca gttgctggcc 3180

aagagcggcc cggtgaaggc cgacgccgaa gaagtggctg acgccgaagc cgattaa 3237

<210> 14
<211> 3174
<212> DNA
<213> Homo sapiens

<400> 14
atggcaatgc agatgcagct tgaagcaaat gcagatactt cagtggaaga agaaagcttt 60

ggcccacaac ccatttcacg gttagagcag tgtggcataa atgccaacga tgtgaagaaa 120

ttggaagaag ctggattcca tactgtggag gctgttgcct atgcgccaaa gaaggagcta 180

ataaatatta agggaattag tgaagccaaa gctgataaaa ttctggctga ggcagctaaa 240

ttagttccaa tgggtttcac cactgcaact gaattccacc aaaggcggtc agagatcata 300

cagattacta ctggctccaa agagcttgac aaactacttc aaggtggaat tgagactgga 360

tctatcacag aaatgtttgg agaattccga actgggaaga cccagatctg tcatacgcta 420

gctgtcacct gccagcttcc cattgaccgg ggtggaggtg aaggaaaggc catgtacatt 480

gacactgagg gtacctttag gccagaacgg ctgctggcag tggctgagag gtatggtctc 540

tctggcagtg atgtcctgga taatgtagca tatgctcgag cgttcaacac agaccaccag 600

acccagctcc tttatcaagc atcagccatg atggtagaat ctaggtatgc actgcttatt 660

gtagacagtg ccaccgccct ttacagaaca gactactcgg gtcgaggtga gctttcagcc 720

aggcagatgc acttggccag gtttctgcgg atgcttctgc gactcgctga tgagtttggt 780

gtagcagtgg taatcactaa tcaggtggta gctcaagtgg atggagcagc gatgtttgct 840

gctgatccca aaaaacctat tggaggaaat atcatcgccc atgcatcaac aaccagattg 900

tatctgagga aaggaagagg ggaaaccaga atctgcaaaa tctacgactc tccctgtctt 960

cctgaagctg aagctatgtt cgccattaat gcagatggag tgggagatgc caaagacgag 1020

ctcggagaag gtcaaggaca gggacaaggt ccaggacgag gatacgcata taagcttatg 1080

gcaatgcaga tgcagcttga agcaaatgca gatacttcag tggaagaaga aagctttggc 1140

ccacaaccca tttcacggtt agagcagtgt ggcataaatg ccaacgatgt gaagaaattg 1200

gaagaagctg gattccatac tgtggaggct gttgcctatg cgccaaagaa ggagctaata 1260

aatattaagg gaattagtga agccaaagct gataaaattc tggctgaggc agctaaatta 1320

gttccaatgg gtttcaccac tgcaactgaa ttccaccaaa ggcggtcaga gatcatacag 1380

attactactg gctccaaaga gcttgacaaa ctacttcaag gtggaattga gactggatct 1440

atcacagaaa tgtttggaga attccgaact gggaagaccc agatctgtca tacgctagct 1500

gtcacctgcc agcttcccat tgaccggggt ggaggtgaag gaaaggccat gtacattgac 1560

actgagggta cctttaggcc agaacggctg ctggcagtgg ctgagaggta tggtctctct 1620

ggcagtgatg tcctggataa tgtagcatat gctcgagcgt tcaacacaga ccaccagacc 1680

cagctccttt atcaagcatc agccatgatg gtagaatcta ggtatgcact gcttattgta 1740

gacagtgcca ccgcccttta cagaacagac tactcgggtc gaggtgagct ttcagccagg 1800

cagatgcact tggccaggtt tctgcggatg cttctgcgac tcgctgatga gtttggtgta 1860

gcagtggtaa tcactaatca ggtggtagct caagtggatg gagcagcgat gtttgctgct 1920

gatcccaaaa aacctattgg aggaaatatc atcgcccatg catcaacaac cagattgtat 1980

ctgaggaaag gaagagggga aaccagaatc tgcaaaatct acgactctcc ctgtcttcct 2040

gaagctgaag ctatgttcgc cattaatgca gatggagtgg gagatgccaa agacggatcc 2100

ggagaaggtc aaggacaggg acaaggtcca ggacgaggat acgcatatgc atgcatggca 2160

atgcagatgc agcttgaagc aaatgcagat acttcagtgg aagaagaaag ctttggccca 2220

caacccattt cacggttaga gcagtgtggc ataaatgcca acgatgtgaa gaaattggaa 2280

gaagctggat tccatactgt ggaggctgtt gcctatgcgc caaagaagga gctaataaat 2340

attaagggaa ttagtgaagc caaagctgat aaaattctgg ctgaggcagc taaattagtt 2400

ccaatgggtt tcaccactgc aactgaattc caccaaaggc ggtcagagat catacagatt 2460

actactggct ccaaagagct tgacaaacta cttcaaggtg gaattgagac tggatctatc 2520

acagaaatgt ttggagaatt ccgaactggg aagacccaga tctgtcatac gctagctgtc 2580

acctgccagc ttcccattga ccggggtgga ggtgaaggaa aggccatgta cattgacact 2640

gagggtacct ttaggccaga acggctgctg gcagtggctg agaggtatgg tctctctggc 2700

agtgatgtcc tggataatgt agcatatgct cgagcgttca acacagacca ccagacccag 2760

ctcctttatc aagcatcagc catgatggta gaatctaggt atgcactgct tattgtagac 2820

agtgccaccg ccctttacag aacagactac tcgggtcgag gtgagctttc agccaggcag 2880

atgcacttgg ccaggtttct gcggatgctt ctgcgactcg ctgatgagtt tggtgtagca 2940

gtggtaatca ctaatcaggt ggtagctcaa gtggatggag cagcgatgtt tgctgctgat 3000

cccaaaaaac ctattggagg aaatatcatc gcccatgcat caacaaccag attgtatctg 3060

aggaaaggaa gaggggaaac cagaatctgc aaaatctacg actctccctg tcttcctgaa 3120

gctgaagcta tgttcgccat taatgcagat ggagtgggag atgccaaaga ctga 3174

<210> 15
<211> 34
<212> DNA
<213> Artificial Sequence

<220>
<223> MS2-derived stem-loop for binding

<400> 15
ggccaacatg aggatcaccc atgtctgcag ggcc 34

<210> 16
<211> 25
<212> DNA
<213> Artificial Sequence

<220>
<223> PP7-derived stem-loop for binding

<400> 16
taaggagttt atatggaaac cctta 25

<210>  17
<211>  17
<212>  DNA
<213>  Artificial Sequence

<220>
<223>  P22-derived stem-loop for binding B-box

<400>  17
accgccgaca acgcggt                                                    17


<210>  18
<211>  385
<212>  DNA
<213>  Bacteriophage MS2

<400>  18
atggcttcaa actttactca gttcgtgctc gtggacaatg gtgggacagg ggatgtgaca      60

gtggctcctt ctaatttcgc taatggggtg gcagagtgga tcagctccaa ctcacggagc     120

caggcctaca aggtgacatg cagcgtcagg cagtctagtg cccagaagag aaagtatacc     180

atcaaggtgg aggtccccaa agtggctacc cagacagtgg gcggagtcga actgcctgtc     240

gccgcttgga gatcctacct gaacatggaa ctcactatcc caattttcgc taccaattct     300

gactgtgaac tcatcgtgaa ggcaatgcag gggctcctca aagacggcaa tcctatccct     360

tccgccatcg ccgctaactc aggca                                           385


<210>  19
<211>  387
<212>  DNA
<213>  Bacteriophage PP7

<400>  19
atgtccaaaa ccatcgttct ttcggtcggc gaggctactc gcactctgac tgagatccag      60

tccaccgcag accgtcagat cttcgaagag aaggtcgggc ctctggtggg tcggctgcgc     120

ctcacggctt cgctccgtca aaacggagcc aagaccgcgt atcgagtcaa cctaaaactg     180

gatcaggcgg acgtcgttga ttgctccacc agcgtctgcg gcgagcttcc gaaagtgcgc     240

tacactcagg tatggtcgca cgacgtgaca atcgttgcga atagcaccga ggcctcgcgc     300

aaatcgttgt acgatttgac caagtccctc gtcgcgacct cgcaggtcga agatcttgtc     360

gtcaaccttg tgccgctggg ccgttaa                                         387


<210>  20
<211>  303
<212>  DNA
<213>  Bacteriophage P22

<400>  20
atgacggtta tcacctacgg gaagtcaacg tttgcaggca atgctaaaac tcgccgtcat      60

gagcggcgca gaaagctagc catagagcgc gacaccatct gcaatatcat cgattcaatt 120

tttggctgcg atgctcctga tgcttctcag gaagttaaag ccaaaagaat tgaccgtgtc 180

accaaagcca tttcgcttgc cggaacgcgt cagaaggaag ttgaaggagg atctgtactt 240

cttccaggcg tagcacttta cgcggctggt catcgtaaga gcaaacaaat aacagcgagg 300

taa 303

<210> 21
<211> 2753
<212> DNA
<213> Artificial Sequence

<220>
<223> MSH2 dominant-negative allele gene sequence

<400> 21
atggctgggt taaggcagga tcttagacag catctgaagc gaatctcaga tgttgagagg 60

cttttgcgca gtctcgagag aagaagaggt gggttacagc acattattaa actctatcag 120

gtactttccg cacttcaatc tgcttctctc aatgttaaca aaattgcatt ttcattgtcc 180

taaatgtgtt tatgcaactc tgaagttata ggtatgttat taagttcatt actaattaag 240

tcttcatctt ttctctgcag tcagctataa ggcttccctt catcaaaaca gctatgcaac 300

agtacaccgg agaattcgca tcactcatca gcgagaggta cctgaaaaag cttgaggctt 360

tatcagatca agatcacctt ggaaagttca tcgatttggt tgagtgctct gtagatcttg 420

accagctaga aaatggagaa tacatgatat cttcaaacta cgacaccaaa ttggcatctc 480

tgaaagatca gaaagaattg ctggagcagc agattcacga attgcacaaa aagacagcga 540

tagaacttga tcttcaggtc gacaaggctc ttaaacttga caaagcagcg caatttgggc 600

atgtcttcag gatcacgaag aaggaagagc caaagatcag gaagaagctg acgacacagt 660

ttatagtgct ggagactcgc aaagacggag tgaagttcac aaacacaaag ctaaaaaaac 720

tgggcgacca gtaccaaagt gttgtggatg attataggag ctgtcaaaag gagctcgttg 780

atcgtgtagt tgagactgtt accagcttct ctgaggtatg tttagttatt catattaagc 840

attggactgt tacagaattg gttgtttaaa atcatagtaa actatatgtg gaatttatat 900

gtatattgta tggttatagg tatttgagga cttagctggg ttactttctg aaatggatgt 960

tttgttaagc tttgctgatt tggctgccag ttgccctact ccatactgta ggccagaaat 1020

cacctctttg gttagtacaa tctcaagttg attattttgt tctgaaaatg aatagttttt 1080

tctttccaag tttatgacat aatgttgaga gcacggttaa taaattgtag gatgctggag 1140

atattgtact agaaggaagc agacatccat gtgtagaagc tcaagattgg gtgaatttca 1200

taccaaatga ttgcagactc gtaagtattg aatgtggtaa ataaactgag acgtctttgt 1260

ttttcttgtt tcccttttga cttgaacaaa tacttgtttg ccctttactg ttctttgaaa 1320

tcagatgaga gggaagagtt ggtttcaaat agtaacaggg cctaacatgg gagataagtc 1380

cactttcatc cgccaggtat gatgatttcc tctagttcag ttttgcttca tagacgtatg 1440

actaaagtcg gtttccggcc attataaatc ccaggttggt gtgattgtgc tgatggctca 1500

agttggttcc tttgttcctt gtgataaagc atcaatttcc ataagagact gcatctttgc 1560

ccgtgtagga gcaggcgatt gccaagtgag tttaagttta gccctcaatg aacgaaaaac 1620

tgctgatatc ctgaacaccc ttattccaac tttttttcct ttggtgtgtt agctgcgtgg 1680

agtgtcaact tttatgcaag aaatgcttga aaccgcatcg atattgaaag gcgctactga 1740

taagtcactg ataattatcg atgaacttgg tcgtggaaca tcaacttatg atggttttgg 1800

ttagtttctc tgcaatttct cttctttcat ttggatgttt ttagtaagtt ttctattata 1860

tattcatttt tatggtcata tgtgagattt cagtgctctt gacatcatcg tggtgaatat 1920

atcaggttta gcttgggcta tatgtgagca tctggttcaa gtgaaaagag caccaactct 1980

gtttgctact cacttccatg aacttactgc cttggctcaa gcaaactctg aggtctctgg 2040

taacactgtt ggtgtggcaa acttccatgt cagcgctcac attgacactg aaagccgcaa 2100

actcaccatg ctttacaagg tctggtttat aaattaaaaa attgctgatc tgttgcagtt 2160

aaaagtgtct ctgtttttat gtttaatcta aattacttat ttgattttct tacaaagatg 2220

aaattgaaat taattttgtg tggtgtgttg tttgtctggt taggttgaac caggggcctg 2280

tgaccagagc tttgggattc atgtggcgga atttgccaac ttccctgaaa gcgtcgtggc 2340

cctcgcaaga gagaaagctg cagagctgga agatttctct ccctcctcga tgataatcaa 2400

caatgaggtc ttgattcatt tccccctttg tttttggttg atgatggaat cattctatca 2460

ttcacccatt ctgcagttta tgctatatta ttataaatct atgtgacaaa gatttaattc 2520

tcgtattgtt gtttgcagga gagtgggaag agaaagagca gagaagatga tccagatgaa 2580

gtatcaagag gggcagagcg agctcacaag tttctgaaag agtttgcagc gatgccactt 2640

gataaaatgg agcttaaaga ttcacttcaa cgggtacgtg agatgaaaga tgagctagag 2700

aaagatgctg cagactgcca ctggctcagg cagtttctgt gaagaacccc tga 2753

## Description

The present invention relates to methods, reagents and compositions for providing more accurate and reliable genetic modification. The invention further provides methods, reagents and compositions for in vivo genetic modification of the genome of a human or animal cell. Furthermore, the present invention relates to uses of the said methods, reagents and compositions in the treatment of disease and production of transgenic animals.

In recent times genetic modification by way of random mutagenesis has given way to directed mutagenesis of particular nucleotide sequences using sequence-specific protein complexes.

Examples of such protein complexes include zinc-finger nucleases (ZFN), transcription activator-like effector nucleases (TALENs), complexes derived from the CRISPR-Cas9 system of *Streptococcus pyrogenes* and other bacteria, and CRISPR-Cpf1.

ZFNs and TALENs are both protein nucleases whose protein structure allows them to interact with and recognise a particular DNA sequence before cutting the DNA at a defined location. Thus cutting a particular DNA sequence requires a uniquely designed ZFN or TALEN protein.

In contrast, the CRISPR-Cas9 and CRISPR-Cpf1 systems use a single protein whose activity is directed by an RNA cofactor whose nucleotide sequence defines the location that the endonuclease will act at to produce a double strand break.

Thus, all of these protein complexes act by making a DNA double strand break at a predefined DNA sequence. This double strand break is then normally repaired by the non-homologous end joining (NHEJ) pathway.

Repair by NHEJ is highly efficient and rapid but is more error-prone than the alternative pathway for repair of DNA double-stranded breaks which is homology-directed repair (HDR). Consequently, a proportion of NHEJ pair of events will cause insertion or deletion of nucleotides at the break site. Such insertion or deletion events are known as 'indels'.

Alternatively, larger genetic modifications are enabled by the presence of a donor DNA molecule in the vicinity of an artificially-created DNA double-stranded break. In this instance HDR of the induced DSB causes repair of the DSB with using the sequence of the donor molecule. In this way specific modifications can be made and short sequence insertions are also possible. One example of such a donor and a vector for producing large amounts of such donor molecules is disclosed in WO/2010/084331.

However, the efficiency of genetic modification using this approach is low because most repair of double strand breaks proceeds via the more rapid NHEJ pathway.

Furthermore, while the above-mention protein complexes are directed to specific sequences their endonuclease activity has been known to act at other sites. Such "off-site breaks" are particularly a problem as NHEJ is more error prone.

Thus, there exists a need for alternative and preferably improved methods and reagents for sequence-specific modification of nucleic acid sequences and of DNA sequences in particular. Furthermore, there is a need for techniques and reagents that more reliably and efficiently yield the desired genetic modification. Additionally, there is a need for techniques and reagents that reliably allow insertion of longer DNA sequences at a predefined locus.

An object of the present invention is to provide reagents and techniques for using these reagents that allow more reliable, efficient and accurate modification and/or mutation of a target genome at specific loci within the genome.

There are provided herein proteins and protein-nucleic acid complexes that provide improved transformation efficiencies and methods for carrying out such transformations. Furthermore the said methods, reagents and compositions may be used for *in vivo* genetic modification of the genome of a human or animal cell. Furthermore, the present invention relates to uses of the said methods, reagents and compositions in the treatment of disease.

Accordingly, the present invention provides a nucleic acid encoding a first fusion protein comprising a 5' to 3' DNA exonuclease domain and an RNA binding domain.

Functionally significant domains or regions of different proteins or polypeptides may be combined for expression from an encoding nucleic acid as a fusion protein. For example, particularly advantageous or desirable properties of different proteins or polypeptides may be combined in a hybrid protein, such that the resultant expression product, may include fragments of various parent proteins or polypeptides.

In the fusion proteins described herein the domains of the fusion proteins are preferably joined together via linker peptides. The particular choice of linker will depend on the constituent domains of the fusion protein. The suitability and choice of appropriate linker peptides is discussed in Chen et al. (Adv Drug Deliv Rev. 2013; 65(10): 1357-1369).

The first fusion protein may be for transformation of a eukaryotic cell in concert with an RNA-guided endonuclease.

Both Zalatan et al. (Cell (2015) 160, 339-350) and the CRISPRainbow system described initially by Ma et al. (Nat Biotechnol. 2016 APR 18. doi: 10.1038/nbt.3526) utilise a modified sgRNA containing 3' RNA hairpin aptamers that bind uniquely labelled RNA binding proteins (SEQ ID NO: 12). Thus the sgRNA is functionalised so that it can be used to locate fusion proteins comprising binding domains for the aptamers in association with the sgRNA and hence the endonuclease it is associated with.

The action of the first fusion protein may be for inhibition of NHEJ during transformation of a cellular genome so as to promote HDR. The effect of such 5' to 3' resection on DNA double-strand breaks is to suppress religation of DNA breaks (i.e. by blocking NHEJ), by producing a substrate that is less suitable for NHEJ but is significantly more suitable for HDR. Thus the action of the first fusion protein may be for inhibition of NHEJ during transformation of a cellular genome so as to promote HDR.

The exonuclease may be a dsDNA exonuclease. The exonuclease may be Exo1. Exonuclease I (exoI) is a 5' to 3' exonuclease and is involved in recombination, double-strand break repair, the MMS2 error-free branch of the post replication repair (PRR) pathway and DNA mismatch repair. Preferably the exo1 is the exo1 protein from bacteriophage λ (SEQ ID NO: 11). Without wishing to be bound by theory, this enzyme can produce approximately 100-150bp 3' overhangs at dsDNA break sites during methods of the invention.

The invention also provides a nucleic acid encoding a second fusion protein comprising an endonuclease domain fused to a binding domain for an origin of replication.

The endonuclease may cleave a target nucleic acid molecule in a sequence specific manner. The sequence specific cleavage of the nucleic acid molecule may be double or single stranded (including 'nicking' of duplexed nucleic acid molecules. Double stranded cleavage may yield blunt ends or overhanging termini (5'or 3' overhangs). The sequence specific nuclease preferably act as a monomer but may act as a dimer or multimer. For instance a homodimer wherein both monomers make single strand nicks at a target site can yield a double-strand break in the target molecule. Preferably the cleavage event makes a double-stranded break in the target molecule.

Examples of sequence-specific endonucleases include zinc-finger nucleases (ZFN), transcription activator-like effector nucleases (TALENs), complexes derived from the CRISPR-Cas9 system of *Streptococcus pyrogenes* and other bacteria, and CRISPR-Cpf1.

A nucleic acid molecule may comprise double- or single-stranded DNA or RNA. The nucleic acid molecule may also comprise a DNA-RNA duplex. Preferably the nucleic acid molecule is double-stranded DNA. Preferably the cleavage event makes a double-stranded DNA break in the target molecule.

Preferably the endonuclease is a DNA endonuclease and most preferably this is Cas9. This may be Cas9 from *Streptococcus pyrogenes* or a homologous or functionally equivalent enzyme from another bacteria.

The fusion protein may comprise an endonuclease and a component of the replication initiation complex or replication complex.

The components of the replication initiation complex or replication complex are necessarily associated with origins of replication and may be covalently attached thereto or to the elongating nucleic acid molecule. Suitably the origin of replication is derived from a virus. Most suitably the virus is circovirus or another member of the circoviridae, i.e. the genera circovirus and cyclovirus. Preferably the virus is porcine circovirus. However, members of the circoviridae are found in a large number of bird and mammal hosts.

The use of porcine circovirus is advantageous because this virus is non-pathogenic in humans.

Circovirus *Rep* protein (GV-Rep) binds to the circovirus origin of replication and thus becomes covalently linked to the ssDNA strand of donor DNA produced by rolling circle replication initiated at the origin of replication. Thus the newly replicated donor DNA molecule is covalently linked to the second fusion protein and is necessarily brought into close proximity to the site of the double-stranded DNA break caused by the endonuclease.

The invention also provides a nucleic acid encoding a third fusion protein comprising a recombination inducing domain and an RNA binding domain.

The recombination domain may be a protein or polypeptide that interacts with a target or donor nucleic acid molecule in order to catalyse modification of the nucleotide sequence of the target nucleic acid with reference to the nucleotide sequence of the donor nucleic acid molecule.

Modification of the target nucleic acid may be by way of insertion of all or a part of the sequence of the donor nucleic acid molecule or substitution of all or a part of the sequence of the donor nucleic acid molecule for a homologous section of the target nucleic acid molecule. In this way deletions, insertions, frameshift mutations and single nucleotide mutations may be achieved.

The recombination inducing event caused or mediated by the recombination inducing domain may be initiating or catalysing strand exchange between the target and donor nucleic acid molecules.

The recombination domain may be RecA from *E. coli* or a homologue thereof, Rad51 or a homologue thereof from a plant or another organism, or an annealase from such as bacteriophage λ recombination protein *beta* (BET; Redβ) or a homologue thereof. Studies of phage lambda *in vivo* have indicated that bacteriophage λ beta protein can catalyse steps that are central to both the strand annealing and strand invasion pathways of recombination. A homologous protein in this case may have functional or sequence homology, preferably functional homology.

Preferably the recombination domain is a trimer of RecA (SEQ ID NO: 13) or Rad51 (SEQ ID NO: 14) monomers. Most preferably the monomers are joined by peptide linkers. Use of a trimer of monomers for the recombination domain is advantageous because this allows binding of a turn of the nucleic acid helix in order to more efficiently initiate strand exchange and hence HDR

The invention also provides a nucleic acid encoding a fourth fusion protein comprising a domain comprising an inhibitor of the mismatch repair pathway; and an RNA binding domain.

MSH2 and MSH6 are proteins involved in base mismatch repair and the repair of short insertion/deletion loops. The MSH2 dominant-negative mutant (SEQ ID NO: 21) competes with MSH2 binding to mismatches thus blocking the ability of the wild-type MSH2 protein to repair these mismatches. A dominant negative allele of MSH6 is also known and may be used in the same way as the dominant negative allele of MSH2.

The RNA binding domains of any of the first, third and fourth fusion proteins may bind to the RNA component of an RNA-guided endonuclease for use in transformation mediated by the RNA-guided endonuclease. Preferably a RNA component is a tracrRNA molecule or domain for use in transformation using the CRISPR-Cas9 system.

The invention also provides a method of transforming the genome of a human or animal cell comprising the steps of:
a. expressing an RNA-guided endonuclease in the cell;
b. expressing in the cell or introducing into the cell a sequence specific guide RNA to direct cleavage by the endonuclease domain to a specific locus
c. expressing in the cell the nucleic acid encoding the first fusion protein or introducing the first fusion protein into the cell.

Thus the invention provides a system with multiple features that may be used separately or in concert. These features include:
a. Induction of dsDNA break using the sequence-specific endonuclease of the second fusion protein.
b. Delivery of donor nucleic acid molecule to within close proximity of the induced DNA break by associating the donor nucleic acid molecule with the origin-binding domain of the second fusion protein.
c. Suppression of religation of DNA breaks (i.e. blocking NHEJ), preferably, as noted above, by 5' to 3' resection of double-stranded DNA breaks in order to produce a substrate that is not suitable for NHEJ but is more suitable for HDR;
d. Delivery of recombinase to the induced dsDNA break.
e. Suppression of the mismatch repair pathway in the vicinity of the induced dsDNA breaks by providing an inhibitor of this pathway. As noted above this is preferably a fusion protein comprising a dominant negative suppressor protein of the mismatch repair system.
Features c, d, and e are supplied to the HDR complex by their being provided in the form of the first, third and fourth fusion proteins, i.e. each comprises a domain that binds to an aptamer engineered to be part of the sgRNA that guides the endonuclease activity of the first fusion protein.

The first, third and fourth fusion proteins each comprises a domain that binds to an aptamer engineered to be part of the sgRNA that guides the endonuclease activity of an RNA-guided endonuclease. Therefore the first, third and fourth fusion proteins may be used in concert with an RNA-guided endonuclease other than the second fusion protein, such as Cas9 or Cpf1.

One advantage flowing from use of any or all of the first, second, third and/or fourth fusion proteins of the invention is more reliable and efficient genetic modification.

A further advantage is that use of any or all of the first, second, third and/or fourth fusion proteins of the invention allows for insertion of longer DNA sequences at a locus or loci acted on by a sequence-guided endonuclease than has previously been reported.

The invention also provides a method of modifying the genome of a human or animal, or human or animal cell comprising:
a. expressing in the cell the nucleic acid encoding the first fusion protein or introducing the first fusion protein into the cell; and
b. expressing in the cell or introducing into the cell a donor nucleic acid molecule comprising an origin of replication.

An animal in the context of the present disclosure may by any multicellular vertebrate or invertebrate animal. Suitably, the animal is a model organism used for biological, physiological or genetic research. Accordingly the animal may be selected from: mouse (*Mus musculus*), zebrafish (*Danio rerio*), fruit fly (*Drosophila melanogaster*), cat (*Felis sylvestris catus*), chicken (*Gallus gallus*), dog (*Canis lupus familiaris*), guinea pig (*Cavia porcellus*), rat (*Rattus norvegicus*) and nematode worm (*Caenorhabditis elegans*),

Suitably, the animal is a domesticated or farmed animal. Accordingly the animal may be selected from: goat (*Capra aegagrus hircus*), pig (*Sus scrofa domesticus*), sheep (*Ovis aries*), cattle (*Bos taurus*), cat (*Felis catus*), donkey (*Equus africanus asinus*), duck (*Anas platyrhynchos domesticus*), water buffalo, including "river buffalo" (*Bubalus bubalis bubalis*) and "swamp buffalo" (*Bubalus bubalis carabenesis*), Western honey bee (*Apis mellifera*), including subspecies Italian bee (*A. mellifera ligustica*), European dark bee (*A. mellifera mellifera*), Carniolan honey bee (*A. mellifera carnica*) and Caucasian honey bee (*A. mellifera caucasia*), Greek bee (*A. mellifera cecropia*), dromedary camel (*Camelus dromedarius*), horse (*Equus ferus caballus*), silkmoth (*Bombyx mori*), pigeon (*Columba livia a*), goose (*Anser domesticus and Anser cygnoides domesticus*), yak (*Bos grunniens*), bactrian camel (*Camelus bactrianus*), llama (*Lama glama*), alpaca (*Vicugna pacos*), guineafowl (*Numida meleagris*), ferret (*Mustela putorius furo*), turkey (*Meleagris gallopavo*) grass carp, silver carp, common carp, nile tilapia, bighead carp, catla (indian carp), crucian carp, atlantic salmon, roho labeo, milkfish, rainbow trout, wuchang bream, black carp, northern snakehead and amur catfish.

The donor nucleic acid molecule may comprise:
a. a donor nucleic acid sequence;
b. flanking nucleic acid sequences located 5' and 3' to the donor nucleic acid sequence;
c. an origin of replication 5' to the 5' flanking nucleotide sequence, and
d. a replication terminator 3' to the 3' flanking nucleotide sequence.

The flanking nucleic acid sequences located 5' and 3' to the donor nucleic acid sequence may be homologous to loci flanking the target loci, sequence or nucleotide.

The replication terminator may be a non-functioning origin of replication that is still capable of terminating replication when a replication fork reaches it. In a specific example, a circovirus (e.g. from porcine circovirus) origin of replication is nicked by the Rep protein at a particular location on a stem loop characteristic of the origin of replication. As long as the stem loop is present and correctly nicked then replication will be terminated at that location. Other sequence elements of the origin are not essential for termination and therefore can be omitted from the replication terminator in this example.

However, the nick at the replication terminator derived from such an origin of replication (in, for instance circoviruses) may still be competent for relegation of the nicked stem loops at the active origin of replication and the downstream terminator/origin of replication. In this way a nucleic acid circle with an active origin of replication is provided and may be actively replicated by rolling circle replication or another mode of replication.

Rolling circle replication of the donor DNA acid molecule has the advantage of providing a large amount of donor DNA nucleic acid. Provision of a relatively large amount of donor nucleic acid molecule means that the probability of the successful transformation is raised.

The methods described herein may comprise introducing a double strand break into the genome in the presence of an exogenous donor nucleic acid molecule comprising a donor nucleic acid sequence as a template for modifying the genome or as an exogenous sequence to be integrated into the genome and a DNA repair mechanism modifies the genome via homology-directed repair (HDR).

The method may further comprise the step or effect of suppressing non-homologous end joining (NHEJ) repair of a DNA double-strand break to promote repair of the break by HDR by expressing in the cell a nucleic acid encoding the first fusion protein or introducing the first fusion protein into the cell.

The methods described herein may comprise introducing a double strand break into the genome in the presence of an exogenous donor nucleic acid molecule comprising a donor nucleic acid sequence as a template for modifying the genome or as an exogenous sequence to be integrated into the genome and a DNA repair mechanism modifies the genome via homology-directed repair (HDR) the method comprising:
suppressing non-homologous end joining (NHEJ) repair of the break to promote repair by HDR by expressing in the cell a nucleic acid encoding the first fusion protein or introducing the first fusion protein into the cell.

The method may further comprise the steps of:
a. expressing in the cell one or more nucleic acids encoding the second, third and fourth fusion proteins; or
b. introducing into the cell one or more of the second, third and fourth fusion proteins.

The method may further comprise the steps of:
a. expressing in the cell or introducing into the cell a sequence specific guide RNA to direct cleavage by the endonuclease domain to a specific locus; and
b. expressing in the cell one or more nucleic acids of the first, third and fourth fusion proteins or introducing one or more of the first, third and fourth fusion proteins into the cell.

The method may further comprise the step of expressing in the cell two or more nucleic acids encoding the first, third and fourth fusion proteins or introducing into the cell two or more of the first, third and fourth fusion proteins, wherein the RNA binding protein domains of the respective fusion proteins bind to different RNA sequences.

In this way the first fusion protein may be using in concert with the second, third and fourth fusion proteins for transformation of a non-animal cell or organism in concert with an RNA-guided endonuclease.

Expression of the first, second, third and fourth fusion proteins during a method of modifying the genome as describe herein may be via inducible and/or transient expression.

Various methods for introducing nucleic acids encoding the fusion proteins and nucleic acids of the invention are envisaged these include electroporation and infiltration in order to introduce proteins, DNA and/or RNA.

The invention further provides a first fusion protein comprising a 5' to 3' DNA exonuclease domain and an RNA binding domain.

The invention also provides a second fusion protein comprising an endonuclease and a component of the replication initiation complex or replication complex.

The invention also provides a third fusion protein comprising a recombination inducing domain and an RNA binding domain

The invention further provides a fourth fusion protein comprising a domain comprising an inhibitor of the mismatch repair pathway and an RNA binding domain.

The invention further provides for use of the first fusion protein, or a nucleic acid encoding the fusion protein in transformation of a human or animal cell, or human or animal cell line using an RNA-guided endonuclease.

The invention further provides for use of the second fusion protein, or a nucleic acid encoding the second fusion protein in transformation of a non-animal organism or cell.

The invention also provides for use of the first fusion protein, or a nucleic acid encoding the first fusion protein in concert with the second, third and fourth fusion proteins in transforming a non-animal organism or cell using an RNA-guided endonuclease.

The invention further provides vectors comprising the nucleic acids of the invention. Such vectors may be suitable for modification in vitro or in vivo.

Vectors of the invention capable of expressing products encoded on nucleotides of the invention may also be suitable for expression in a host cell or cell-free system. Suitably the host cell may be a cultured plant cell, yeast cell or bacterial cell, e.g. *Escherichia coli.* Compositions and products of the invention may be obtained by methods comprising expressing such encoded products in a suitable host cell or cell-free system.

The invention also provides uses of the methods, reagents and compositions disclosed herein for introducing desirable traits to non-animal organisms or ameliorating or removing non-desirable traits in these organisms. Accordingly, the invention also provides non-animal transgenic organisms, transgenic cells thereof and transgenic non-animal cell lines. Organisms which include a transgenic cell according to the invention are also provided.

The invention further provides methods of treating disease or other conditions of non-animal organisms or cells by utilising the methods, reagents and compositions disclosed herein.

The invention also provides the methods, reagents and compositions disclosed herein for use in the treatment of disease or humans or animals.

The invention also provides uses of the methods, reagents and compositions disclosed herein for introducing desirable genetic characteristics to humans or animals or ameliorating or for removing non-desirable genetic characteristics in humans or animals.

The invention further provides uses of the methods, reagents and compositions disclosed herein for introducing desirable heritable characteristics to non-human animals for ameliorating or for removing non-desirable inherited characteristics in these animals.

Accordingly, the invention also provides non-human transgenic animals, transgenic cells thereof and transgenic human or animal cell lines. Animals which include a transgenic cell according to the invention are also provided.

The invention further provides methods of treating disease or other conditions of humans or animals by utilising the methods, reagents and compositions disclosed herein.

The invention also provides uses of the methods, reagents and compositions disclosed herein for introducing desirable genetic characteristics to human or animal embryonic stem cells and/or stem cell lines or for ameliorating or removing non-desirable genetic characteristics in these stem cells and/or stem cell lines.

The invention further provides uses of the methods, reagents and compositions disclosed herein for therapeutic or diagnostic purposes applied to a human embryo and which are useful to it

The invention is now illustrated in specific embodiments with reference to the accompanying drawings in which:

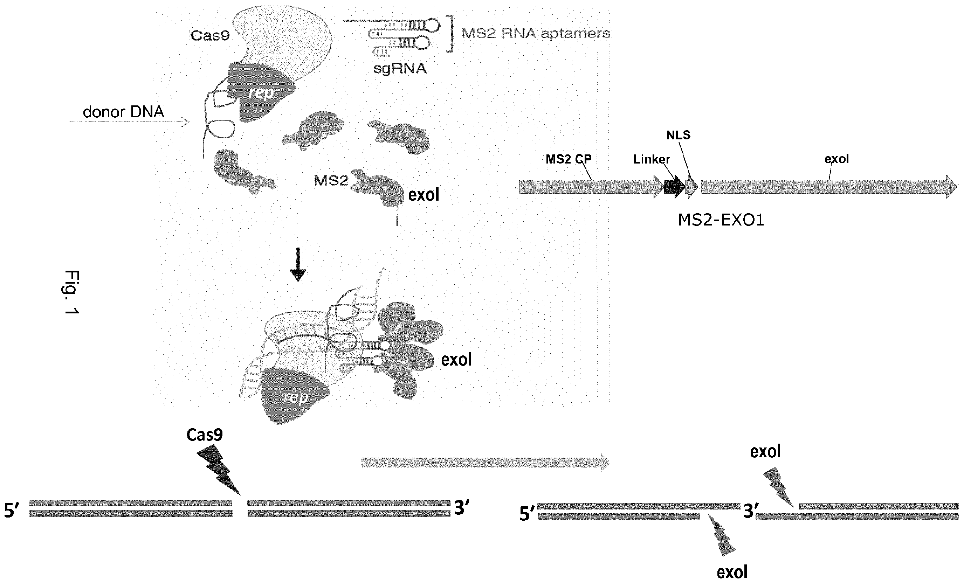

- Fig. 1: shows a schematic representation of inducing a DNA double strand break with Cas9 protein (Cas9) and resecting the DNA DSB with exo1. The exo1-MS2 fusion protein is engineered to bind to the single guide RNA (sgRNA) via aptamer loops on the sgRNA that bind to the MS2 domain (SEQ ID NO: 8).

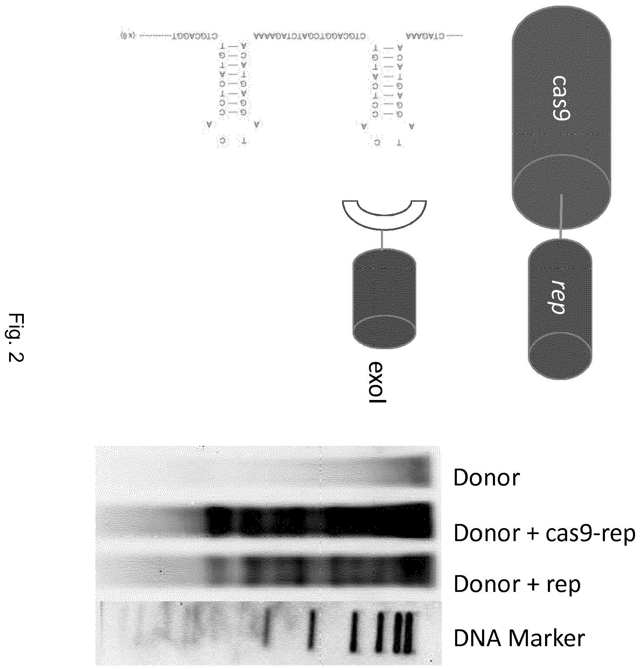

- Fig. 2: shows a schematic representation of a cas9-Rep (virus replication associated protein) fusion protein (SEQ ID NO: 10) and an exo1-MS2 fusion protein showing its binding to an aptamer loop. Also shown is an electrophoresis gel demonstrating the activity of the cas9-Rep fusion protein (SEQ ID NO: 10).

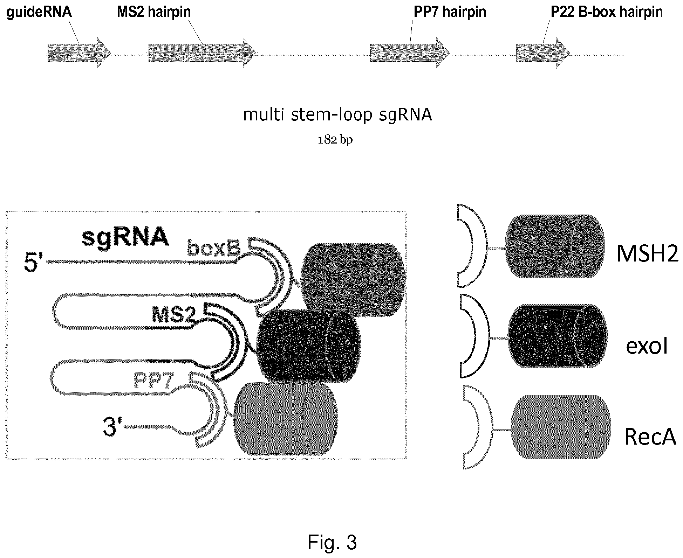

- Fig. 3: shows the design of a multi stem-loop sgRNA with hairpins from different bacteriophages (MS2, PP7 and P22 bacteriophages; (SEQ ID NOs: 12 and 15-17).

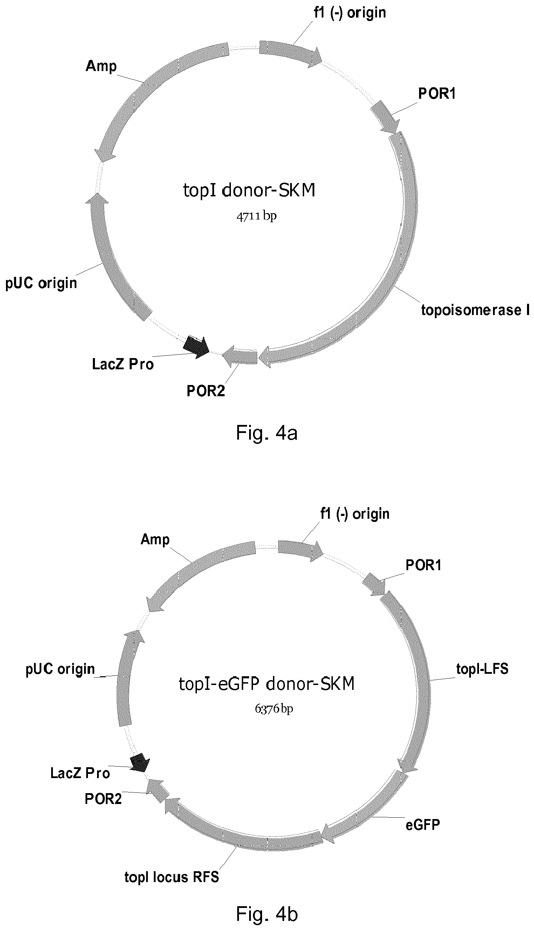

- Fig. 4: shows (a) a donor vector (topI donor-SKM) containing a mutated topoisomerase I gene fragment for generation of resistance to antibiotic camptothecin (SEQ ID NO: 3), and (b) a donor vector (topI-eGFP donor-SKM) comprising a cassette designed for insertion of eGFP into the topoisomerase I locus (SEQ ID NO: 4). POR1 and POR2 (SEQ ID NO: 1) are viral origins of replication from pig circovirus 1 (PCV1)

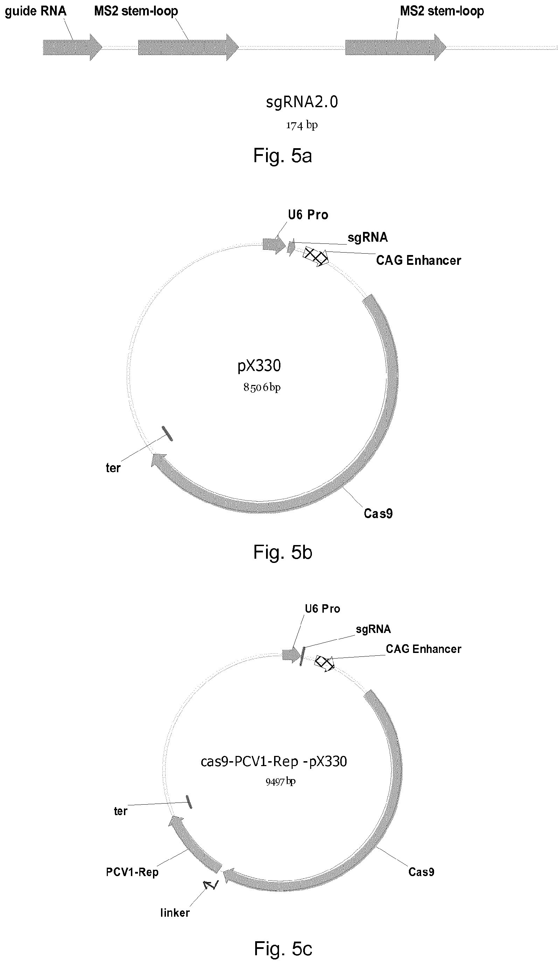

- Fig. 5: shows (a) the design of sgRNA for precise targeting of proteins to the double-stranded DNA break. Two stem-loops from bacteriophage MS2 (SEQ ID NO: 15) were introduced into the sgRNA; (b) a vector containing cas9 (SEQ ID NO: 6) in the pX330 vector as control; and (c) a cas9-PCV1 Rep fusion (SEQ ID NO: 10) cassette for expression in human cells. The constructs also have sgRNAs with a guide sequence for targeting the human topoisomerase I gene.

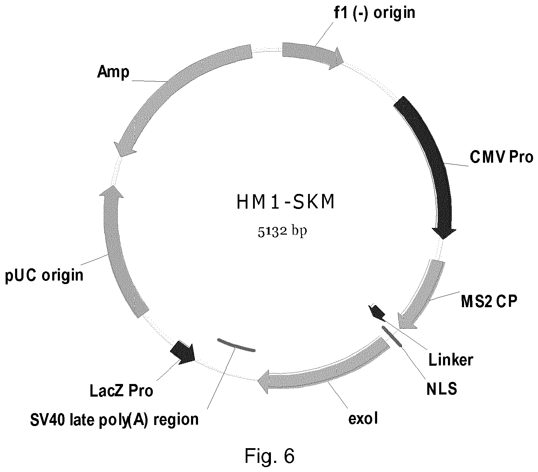

- Fig. 6: shows a vector containing a cassette for translational fusion of the MS2 coat protein (SEQ ID NO: 18) and exonuclease I (SEQ ID NO: 11) to generate targeting of the exoI protein to double-stranded DNA breaks.

### Example 1

### Gene Editing of Topoisomerase I in Human Cells

To assess efficiency of gene targeting in human cells a set of constructs was prepared for targeting topoisomerase I.

Transfections were carried out by calcium phosphate transfection (see Calcium phosphate-mediated transfection of eukaryotic cells, Nature Methods 2005, volume 2, pages 319-320 and kits derived therefrom as available from ThermoFisher Scientific or Merck) but may also be carried out by electroporation (see method below).

A mutant topoisomerase I gene fragment for generation of resistance to antibiotic camptothecin (SEQ ID NO: 3) was introduced into human HEK293 cells (topI donor-SKM; Figure 4a). The resulting clones tested for camptothecin resistance.

**Table 1**

| *Experiment in human HEK293 cells* | *Heterozygous clones* | *Homozygous clones* | *Percentage of homozygous clones* |
|---|---|---|---|
| (i) Donor vector POR12 | 2 out of 47 | 0 out of 47 | 0% |
| TOP1 FtoS sgRNA2.0 cas9 | | | |
| (ii) Donor vector POR12 | 4 out of 47 | 3 out of 47 | 6.3% |
| TOP1 FtoS sgRNA2.0 cas9-PCV1-Rep | | | |
| (iii) Donor vector POR12 | 6 out of 47 | 4 out of 47 | 8.5% |
| TOP1 FtoS sgRNA2.0 cas9-PCV1-Rep MS2CP-ExoI | | | |

These results demonstrate that gene editing mediated by the Cas9-PCV1 Rep fusion (SEQ ID NO: 10) (experiment (ii)) is significantly more efficient than for the control experiment using cas9 alone.

These results also demonstrate that gene editing mediated by the Cas9-PCV1 Rep fusion (SEQ ID NO: 2) and an exo1-MS2 fusion protein designed to bind to the Cas9-sgRNA complex (experiment (iii)) is yet more efficient than either the control experiment using cas9 alone or experiment (ii) using the Cas9-PCV1 Rep fusion alone.

### Example 2

### Insertion of eGFP into the Topoisomerase I locus of Human Cells

Human cells carrying the mutated topoisomerase I gene generated by the method described above were then co-transformed with donor construct topl-eGFP donor-SKM (Fig. 4b) (SEQ ID NO: 4) and a construct carrying expressing (i) Cas9 (construct pX330; Fig. 5b), (ii) Cas9-PCV1Rep fusion (construct cas9-PCV1-Rep-pX330; Fig. 5c) or (iii) Cas9-PCV1 Rep fusion and an exo1-MS2 fusion protein designed to bind to the sgRNA that is in turn bound to the cas9 (construct HM1-SKM; Fig 6).

The cells subsequently generated were assessed for eGFP activity; eGFP activity indicating successful transformation *in vivo* using the gene targeting system. The results of these experiments are set out in Table 2.

**Table 2**

| *Experiment in human HEK293 cells with eGFP* (717bp) | *Heterozygous clones* | *Homozygous clones* | *Percentage of homozygous clones* |
|---|---|---|---|
| (i) Donor vector POR12 | | 0 out of 47 | 0% |
| TOP1 eGFP sgRNA2.0 cas9 | | | |
| (ii) Donor vector POR12 | | 0 out of 47 | 0% |
| TOP1 eGFP sgRNA2.0 cas9-PCV1-Rep | | | |
| (iii) Donor vector POR12 | | 3 out of 47 | 6.3% |
| TOP1 eGFP sgRNA2.0 cas9-PCV1-Rep MS2CP-ExoI | | | |

The transformation being carried out in this instance is a relatively large insertion of 717bp. As noted above, hereto now it has not been possible to insert longer sequences of nucleotides into a locus targeted by RNA-directed mutagenesis.

These results also demonstrate that gene editing mediated by the Cas9-PCV1Rep fusion in concert with an exo1-MS2 fusion protein designed to bind to the Cas9-sgRNA complex (experiment (iii)) allows the efficient insertion of longer nucleotide sequences into the targeted locus. As is also demonstrated such an insertion is not achieved by using cas9 alone or the Cas9-PCV1Rep fusion alone.

### Transformation Method - Electroporation

This protocol was adapted from "DNA transfection by electroporation" in Molecular Cloning: A Laboratory Manual (eds. Sambrook, J. & Russell, D.W.) 16.33-16.36 (Cold Spring Harbor Laboratory Press, Cold Spring Harbor, New York, 2001; http://www.cshlpress.com/link/molclon3.htm).

### Preparation of the cells

1. Collect the cells to be transfected from cultures in the mid- to late-logarithmic phase of growth. Use either a rubber policeman or trypsin to release adherent cells. Centrifuge at 500g at 4 °C for 5 min.
2. Resuspend the cell pellet in 0.5× volume of the original growth medium and measure the cell number using a hemocytometer.
3. Collect the cells by centrifugation, as described in Step 1 and resuspend them in growth medium or phosphate-buffered saline (PBS; 137 mM NaCl, 2.7 mM KCl, 10 mM Na₂HPO₄ and 2 mM KH₂PO₄) at 15-25 °C at a concentration of 2.5 × 10⁶ to 2.5 × 10⁷ cells/ml.
4. Transfer 400-µl aliquots of the cell suspension (10⁶-10⁷ cells) into as many labeled electroporation cuvettes as needed. Place the loaded cuvettes on ice.
5. Set the parameters on the electroporation device. (A typical capacitance value is 1,050 µF.) Voltages range from 200 to 350 V, depending on the cell line, but generally average 260 V. Use an infinite internal resistance value. Discharge a blank cuvette containing PBS at least twice before electroporating cells.

### Introduction of the DNA

6. Add 10-30 µg of plasmid DNA in a volume of up to 40 µl to each cuvette containing cells. (Some investigators add carrier DNA, for example, salmon sperm DNA, to bring the total amount of DNA to 120 µg.) Gently mix the cells and DNA by pipetting the solution up and down. Proceed to Step 7 without delay.
   *Do not introduce air bubbles into the suspension during the mixing step.*
7. Immediately transfer the cuvette to the electroporator and discharge the device. After 1-2 min, remove the cuvette, place it on ice and proceed immediately to the next step.
8. Transfer the electroporated cells to a 35-mm culture dish using a micropipettor equipped with a sterile tip. Rinse out the cuvette with a fresh aliquot of growth medium and add the washings to the culture dish. Transfer the dish to a humidified incubator at 37 °C with an atmosphere of 5-7% CO₂.
9. Repeat Steps 6-8 until all of the DNA cell samples have been treated. Recording the actual pulse time for each cuvette will facilitate comparisons between experiments.
10. If the objective is stable transformation of the cells, proceed directly to Step 11. For transient expression, examine the cells 24-96 h after electroporation using an appropriate assay.
11. To isolate stable transfectants, incubate for 48-72 h in complete medium, trypsinize the cells and replate them in the appropriate selective medium. Change the selective medium every 2-4 d for 2-3 weeks to remove the debris of dead cells and to allow colonies of resistant cells to grow. Thereafter, clone individual colonies and propagate for the appropriate assay.

### Nucleotide Sequences

Pig circovirus 1 (PCV1) origin of replication (POR) (SEQ ID NO: 1) 
Ctgcgggggcgggtccttcttctgcggtaacgcctccttggccacgtcatcctataaaagtgaaagaagtgcgctgctgtagtattaccagcgcactt
cggcagcggcagcacctcggcagcgtcagtgaaaatgccaagcaagaaaagcggcccgcaaccccataagag
PCV1 rep protein (SEQ ID NO: 2) 
atgccaagcaagaaaagcggcccgcaaccccataagaggtgggtgttcacccttaataatccttccgaggaggagaaaaacaaaatacggga
gcttccaatctcccttttgattatttgtttgcggagaggaaggtttggaagagggtagaactcctcacctccaggggtttgcgaattttgctaagaagca
gacttttaacaaggtgaagtggtattttggtgcccgctgccacatcgagaaagcgaaaggaaccgaccagcagaataaagaatactgcagtaaa
gaaggtcacatacttatcgagtgtggagctccgcggaaccaggggaagcgcagcgacctgtctactgctgtgagtacccttttggagacggggtctt
tggtgactgtagccgagcagttccctgtaacgtatgtgagaaatttccgcgggctggctgaacttttgaaagtgagcgggaagatgcagcagcgtga
ttggaagacagctgtacacgtcatagtgggcccgcccggttgtgggaagagccagtgggcccgtaattttactgagcctagcgacacctactggaa
gcctagtagaaataagtggtgggatggatatcatggagaagaagttgttgttttggatgattttatggctggttaccttgggatgatctactgagactgtg
tgaccggtatccattgactgtagagactaaaggcggtactgttcctttttggcccgcagtattttgattaccagcaatcaggcccccccaggaatggtact
cctcaactgctgtcccagctgtagaagctctctatcggaggattactactttgcaattttggaagactgctggagaacaatccacggaggtacccgaa
ggccgatttgaagcagtggacccaccctgtgcccttttcccatataaaataaattactga
topoisomerase I donor (SEQ ID NO: 3) 
gtccggcagagagctgtagccctgtacttcatcgacaagcttgctctgagagcaggcaatgaaaaggaggaaggagaaacagcggacactgtg
ggctgctgctcacttcgtgtggagcacatcaatctacacccagagttggatggtcaggaatatgtggtagagtttgacttcctcgggaaggactccatc
agatactataacaaggtccctgttgagaaacgagtttttaagaacctacaactatttatggagaacaagcagcccgaggatgatctttttgatagactc
aatactggtattctgaataagcatcttcaggatctcatggagggcttgacagccaaggtattccgtacgtacaatgcctccatcacgctacagcagca
gctaaaagaactgacagccccggatgagaacatcccagcgaagatcctttcttataaccgtgccaatcgagctgttgcaattctttgtaaccatcaga
gggcaccaccaaaaacttttgagaagtctatgatgaacttgcaaactaagattgatgccaagaaggaacagctagcagatgcccggagagacct
gaaaagtgctaaggctgatgccaaggtcatgaaggatgcaaagacgaagaaggtagtagagtcaaagaagaaggctgttcagagactggagg
aacagttgatgaagctggaagttcaagccacagaccgagaggaaaataaacagattgccctgggaacctccaaactcagctatctggaccctag
gatcacagtggcttggtgcaagaagtggggtgtcccaattgagaagatttacaacaaaacccagcgggagaagtttgcctgggccattgacatgg
ctgatgaagactatgagttttagccagtctcaagaggcagagttctgtgaagaggaacagtgtggtttgggaaagatggataaactgagcctcactt
gccctcgtgcctgggggagagaggcagcaagtcttaacaaaccaacatctttgcgaaaagataaacctggagatattataagggagagctgagc
cagttgtcctatggacaacttatttaaaaatatttcagatatcaaaattctagctgtatgatttgttttgaattttgtttttattttcaagagggcaagtggatggg
aatttgtcagcgttctaccaggcaaattcactgtttcactgaaatgtttggattctcttagctactgtatgcaaagtccgattatattggtgcgtttttacagtta
gggttttgcaataacttctatattttaatagaaataaattcctaaactcccttccctctctcccatttcaggaatttaaaattaagtagaacaaaaaccca
gcgcacctgttagagtcgtcactctctattgtcatggggatcaattttcattaaacttgaagcagtcgtggctttggcagtgttttggttcagacacctgttca
cagaaaaagcatgatgggaaaatatttcc
Topoisomerase I wtih eGFP insertion donor gene (SEQ ID NO: 4) 
cagcggacactgtgggctgctgctcacttcgtgtggagcacatcaatctacacccagagttggatggtcaggaatatgtggtagagtttgacttcctcg
ggaaggactccatcagatactataacaaggtccctgttgagaaacgagtttttaagaacctacaactatttatggagaacaagcagcccgaggatg
atctttttgatagactcaatactggtattctgaataagcatcttcaggatctcatggagggcttgacagccaaggtattccgtacgtacaatgcctccatc
acgctacagcagcagctaaaagaactgacagccccggatgagaacatcccagcgaagatcctttcttataaccgtgccaatcgagctgttgcaatt
ctttgtaaccatcagagggcaccaccaaaaacttttgagaagtctatgatgaacttgcaaactaagattgatgccaagaaggaacagctagcagat
gcccggagagacctgaaaagtgctaaggctgatgccaaggtcatgaaggatgcaaagacgaagaaggtagtagagtcaaagaagaaggctg
ttcagagactggaggaacagttgatgaagctggaagttcaagccacagaccgagaggaaaataaacagattgccctgggaacctccaaactca
gctatctggaccctaggatcacagtggcttggtgcaagaagtggggtgtcccaattgagaagatttacaacaaaacccagcgggagaagtttgcct
 
gggccattgacatggctgatgaagactatgagtttaagggcgaggagctgttcaccggggtggtgcccatcctggtcgagctggacggcgacgtaa
acggccacaagttcagcgtgtccggcgagggcgagggcgatgccacctacggcaagctgaccctgaagttcatctgcaccaccggcaagctgc
ccgtgccctggcccaccctcgtgaccaccctgacctacggcgtgcagtgcttcagccgctaccccgaccacatgaagcagcacgacttcttcaagt
ccgccatgcccgaaggctacgtccaggagcgcaccatcttcttcaaggacgacggcaactacaagacccgcgccgaggtgaagttcgagggcg
acaccctggtgaaccgcatcgagctgaagggcatcgacttcaaggaggacggcaacatcctggggcacaagctggagtacaactacaacagc
cacaacgtctatatcatggccgacaagcagaagaacggcatcaaggtgaacttcaagatccgccacaacatcgaggacggcagcgtgcagctc
gccgaccactaccagcagaacacccccatcggcgacggccccgtgctgctgcccgacaaccactacctgagcacccagtccgccctgagcaa
agaccccaacgagaagcgcgatcacatggtcctgctggagttcgtgaccgccgccgggatcactctcggcatggacgagctatacaagccagtct
caagaggcagagttctgtgaagaggaacagtgtggtttgggaaagatggataaactgagcctcacttgccctcgtgcctggggagagaggcag
caagtcttaacaaaccaacatctttgcgaaaagataaacctggagatattataagggagagctgagccagttgtcctatggacaacttatttaaaaat
atttcagatatcaaaattctagctgtatgatttgttttgaattttgtttttattttcaagagggcaagtggatgggaatttgtcagcgttctaccaggcaaattca
ctgtttcactgaaatgtttggattctcttagctactgtatgcaaagtccgattatattggtgcgtttttacagttagggttttgcaataacttctatattttaataga
aataaattcctaaactcccttccctctctcccatttcaggaatttaaaattaagtagaacaaaaaacccagcgcacctgttagagtcgtcactctctattg
tcatggggatcaattttcattaaacttgaagcagtcgtggctttggcagtgttttggttcagacacctgttcacagaaaaagcatgatgggaaaatatttc
ctgacttgagtgttcctttttaaatgtgaatttttttttttttaattattttaaaatatttaaaccttttcttgatcttaaagatcgtgtagattggggttggggaggg
atgaagggcgagtgaatctaaggataatgaaataatcagtgactgaaaccattttcccatcatcctttgttctg
CMV promoter (SEQ ID NO: 5) 
caatcccacaaaaatctgagcttaacagcacagttgctcctctcagagcagaatcgggtattcaacaccctcatatcaactactacgttgtgtataac
ggtccacatgccggtatatacgatgactggggttgtacaaaggcggcaacaaacggcgttcccggagttgcacacaagaaatttgccactattaca
gaggcaagagcagcagctgacgcgtacacaacaagtcagcaaacagacaggttgaacttcatccccaaaggagaagctcaactcaagccca
agagctttgctaaggccctaacaagcccaccaaagcaaaaagcccactggctcacgctaggaaccaaaaggcccagcagtgatccagcccca
aaagagatctcctttgccccggagattacaatggacgatttcctctatctttacgatctaggaaggaagttcgaaggtgaagtagacgacactatgttc
accactgataatgagaaggttagcctcttcaatttcagaaagaatgctgacccacagatggttagagaggcctacgcagcaggtctcatcaagacg
atctacccgagtaacaatctccaggagatcaaataccttcccaagaaggttaaagatgcagtcaaaagattcaggactaattgcatcaagaacac
agagaaagacatatttctcaagatcagaagtactattccagtatggacgattcaaggcttgcttcataaaccaaggcaagtaatagagattggagtct
ctaaaaaggtagttcctactgaatctaaggccatgcatggagtctaagattcaaatcgaggatctaacagaactcgccgtgaagactggcgaacag
ttcatacagagtcttttacgactcaatgacaagaagaaaatcttcgtcaacatggtggagcacgacactctggtctactccaaaaatgtcaaagatac
agtctcagaagaccaaagggctattgagacttttcaacaaaggataatttcgggaaacctcctcggattccattgcccagctatctgtcacttcatcga
aaggacagtagaaaaggaaggtggctcctacaaatgccatcattgcgataaaggaaaggctatcattcaagatctctctgccgacagtggtccca
aagatggacccccacccacgaggagcatcgtggaaaaagaagacgttccaaccacgtcttcaaagcaagtggattgatgtgacatctccactga
cgtaagggatgacgcacaatcccactatccttcgcaagacccttcctctatataaggaagttcatttcatttggagaggacacg
cas9 gene (SEQ ID NO: 6) 
atggactataaggaccacgacggagactacaaggatcatgatattgattacaaagacgatgacgataagatggccccaaagaagaagcggaa
ggtcggtatccacggagtcccagcagccgacaagaagtacagcatcggcctggacatcggcaccaactctgtgggctgggccgtgatcaccgac
gagtacaaggtgcccagcaagaaattcaaggtgctgggcaacaccgaccggcacagcatcaagaagaacctgatcggagccctgctgttcgac
agcggcgaaacagccgaggccacccggctgaagagaaccgccagaagaagatacaccagacggaagaaccggatctgctatctgcaagag
atcttcagcaacgagatggccaaggtggacgacagcttcttccacagactggaagagtccttcctggtggaagaggataagaagcacgagcggc
accccatcttcggcaacatcgtggacgaggtggcctaccacgagaagtaccccaccatctaccacctgagaaagaaactggtggacagcaccg
acaaggccgacctgcggctgatctatctggccctggcccacatgatcaagttccggggccacttcctgatcgagggcgacctgaaccccgacaac
agcgacgtggacaagctgttcatccagctggtgcagacctacaaccagctgttcgaggaaaaccccatcaacgccagcggcgtggacgccaag
gccatcctgtctgccagactgagcaagagcagacggctggaaaatctgatcgcccagctgcccggcgagaagaagaatggcctgttcggaaac
ctgattgccctgagcctgggcctgacccccaacttcaagagcaacttcgacctggccgaggatgccaaactgcagctgagcaaggacacctacg
acgacgacctggacaacctgctggcccagatcggcgaccagtacgccgacctgtttctggccgccaagaacctgtccgacgccatcctgctgagc
gacatcctgagagtgaacaccgagatcaccaaggcccccctgagcgcctctatgatcaagagatacgacgagcaccaccaggacctgaccctg
ctgaaagctctcgtgcggcagcagctgcctgagaagtacaaagagattttcttcgaccagagcaagaacggctacgccggctacattgacggcgg
agccagccaggaagagttctacaagttcatcaagcccatcctggaaaagatggacggcaccgaggaactgctcgtgaagctgaacagagagg
acctgctgcggaagcagcggaccttcgacaacggcagcatcccccaccagatccacctgggagagctgcacgccattctgcggcggcaggaa
gatttttacccattcctgaaggacaaccgggaaaagatcgagaagatcctgaccttccgcatcccctactacgtgggccctctggccaggggaaac
agcagattcgcctggatgaccagaaagagcgaggaaaccatcaccccctggaacttcgaggaagtggtggacaagggcgcttccgcccagag
cttcatcgagcggatgaccaacttcgataagaacctgcccaacgagaaggtgctgcccaagcacagcctgctgtacgagtacttcaccgtgtataa
cgagctgaccaaagtgaaatacgtgaccgagggaatgagaaagcccgccttcctgagcggcgagcagaaaaaggccatcgtggacctgctgtt
caagaccaaccggaaagtgaccgtgaagcagctgaaagaggactacttcaagaaaatcgagtgcttcgactccgtggaaatctccggcgtgga
agatcggttcaacgcctccctgggcacataccacgatctgctgaaaattatcaaggacaaggacttcctggacaatgaggaaaacgaggacattc
tggaagatatcgtgctgaccctgacactgtttgaggacagagagatgatcgaggaacggctgaaaacctatgcccacctgttcgacgacaaagtg
atgaagcagctgaagcggcggagatacaccggctggggcaggctgagccggaagctgatcaacggcatccgggacaagcagtccggcaag
acaatcctggatttcctgaagtccgacggcttcgccaacagaaacttcatgcagctgatccacgacgacagcctgacctttaaagaggacatccag
aaagcccaggtgtccggccagggcgatagcctgcacgagcacattgccaatctggccggcagccccgccattaagaagggcatcctgcagaca
gtgaaggtggtggacgagctcgtgaaagtgatgggccggcacaagcccgagaacatcgtgatcgaaatggccagagagaaccagaccaccc
agaagggacagaagaacagccgcgagagaatgaagcggatcgaagagggcatcaaagagctgggcagccagatcctgaaagaacacccc
gtggaaaacacccagctgcagaacgagaagctgtacctgtactacctgcagaatggcgggatatgtacgtggaccaggaactggacatcaac
 
cggctgtccgactacgatgtggaccatatcgtgcctcagagctttctgaaggacgactccatcgacaacaaggtgctgaccagaagcgacaagaa
ccggggcaagagcgacaacgtgccctccgaagaggtcgtgaagaagatgaagaactactggcggcagctgctgaacgccaagctgattaccc
agagaaagttcgacaatctgaccaaggccgagagaggcggcctgagcgaactggataaggccggcttcatcaagagacagctggtggaaacc
cggcagatcacaaagcacgtggcacagatcctggactcccggatgaacactaagtacgacgagaatgacaagctgatccgggaagtgaaagt
gatcaccctgaagtccaagctggtgtccgatttccggaaggatttccagttttacaaagtgcgcgagatcaacaactaccaccacgcccacgacgc
ctacctgaacgccgtcgtgggaaccgccctgatcaaaaagtaccctaagctggaaagcgagttcgtgtacggcgactacaaggtgtacgacgtgc
ggaagatgatcgccaagagcgagcaggaaatcggcaaggctaccgccaagtacttcttctacagcaacatcatgaactttttcaagaccgagatt
accctggccaacggcgagatccggaagcggcctctgatcgagacaaacggcgaaaccggggagatcgtgtgggataagggccgggattttgc
caccgtgcggaaagtgctgagcatgccccaagtgaatatcgtgaaaaagaccgaggtgcagacaggcggcttcagcaaagagtctatcctgccc
aagaggaacagcgataagctgatcgccagaaagaaggactgggaccctaagaagtacggcggcttcgacagccccaccgtggcctattctgtg
ctggtggtggccaaagtggaaaagggcaagtccaagaaactgaagagtgtgaaagagctgctggggatcaccatcatggaaagaagcagcttc
gagaagaatcccatcgactttctggaagccaagggctacaaagaagtgaaaaaggacctgatcatcaagctgcctaagtactccctgttcgagct
ggaaaacggccggaagagaatgctggcctctgccggcgaactgcagaagggaaacgaactggccctgccctccaaatatgtgaacttcctgtac
ctggccagccactatgagaagctgaagggctcccccgaggataatgagcagaaacagctgtttgtggaacagcacaagcactacctggacgag
atcatcgagcagatcagcgagttctccaagagagtgatcctggccgacgctaatctggacaaagtgctgtccgcctacaacaagcaccgggataa
gcccatcagagagcaggccgagaatatcatccacctgtttaccctgaccaatctgggagcccctgccgccttcaagtactttgacaccaccatcgac
cggaagaggtacaccagcaccaaagaggtgctggacgccaccctgatccaccagagcatcaccggcctgtacgagacacggatcgacctgtct
cagctgggaggcgacaaaaggccggcggccacgaaaaaggccggccaggcaaaaaagaaaaagtaa
human U6 promoter (SEQ ID NO: 7) 
Tttcccatgattccttcatatttgcatatacgatacaaggctgttagagagataattggaattaatttgactgtaaacacaaagatattagtacaaaatac
gtgacgtagaaagtaataatttcttgggtagtttgcagttttaaaattatgttttaaaatggactatcatatgcttaccgtaacttgaaagtatttcgatttcttg
gctttatatatcttgtggaaaggac
sgRNA2.0 with MS2 hairpins for modification of topoisomerase I gene (SEQ ID NO: 8) 
gatttacaacaaaacccagcgttttagagctaggccaacatgaggatcacccatgtctgcagggcctagcaagttaaaataaggctagtccgttatc
aacttggccaacatgaggatcacccatgtctgcagggccaagtggcaccgagtcggtgcttttttttttt
SV40 polyA terminator (SEQ ID NO: 9) 
cagacatgataagatacattgatgagtttggacaaaccacaactagaatgcagtgaaaaaaatgctttatttgtgaaatttgtgatgctattgctttatttg
taaccattataagctgcaataaacaagttaacaacaacaattgcattcattttatgtttcaggttcagggggaggtgtgggaggttttttaaagcaagta
aaacctctacaaatgtggta
cas9-B-PCV1 rep fusion protein gene (SEQ ID NO: 10) 
atggactataaggaccacgacggagactacaaggatcatgatattgattacaaagacgatgacgataagatggccccaaagaagaagcggaa
ggtcggtatccacggagtcccagcagccgacaagaagtacagcatcggcctggacatcggcaccaactctgtgggctgggccgtgatcaccgac
gagtacaaggtgcccagcaagaaattcaaggtgctgggcaacaccgaccggcacagcatcaagaagaacctgatcggagccctgctgttcgac
agcggcgaaacagccgaggccacccggctgaagagaaccgccagaagaagatacaccagacggaagaaccggatctgctatctgcaagag
atcttcagcaacgagatggccaaggtggacgacagcttcttccacagactggaagagtccttcctggtggaagaggataagaagcacgagcggc
accccatcttcggcaacatcgtggacgaggtggcctaccacgagaagtaccccaccatctaccacctgagaaagaaactggtggacagcaccg
acaaggccgacctgcggctgatctatctggccctggcccacatgatcaagttccggggccacttcctgatcgagggcgacctgaaccccgacaac
agcgacgtggacaagctgttcatccagctggtgcagacctacaaccagctgttcgaggaaaaccccatcaacgccagcggcgtggacgccaag
gccatcctgtctgccagactgagcaagagcagacggctggaaaatctgatcgcccagctgcccggcgagaagaagaatggcctgttcggaaac
ctgattgccctgagcctgggcctgacccccaacttcaagagcaacttcgacctggccgaggatgccaaactgcagctgagcaaggacacctacg
acgacgacctggacaacctgctggcccagatcggcgaccagtacgccgacctgtttctggccgccaagaacctgtccgacgccatcctgctgagc
gacatcctgagagtgaacaccgagatcaccaaggcccccctgagcgcctctatgatcaagagatacgacgagcaccaccaggacctgaccctg
ctgaaagctctcgtgcggcagcagctgcctgagaagtacaaagagattttcttcgaccagagcaagaacggctacgccggctacattgacggcgg
agccagccaggaagagttctacaagttcatcaagcccatcctggaaaagatggacggcaccgaggaactgctcgtgaagctgaacagagagg
acctgctgcggaagcagcggaccttcgacaacggcagcatcccccaccagatccacctgggagagctgcacgccattctgcggcggcaggaa
gatttttacccattcctgaaggacaaccgggaaaagatcgagaagatcctgaccttccgcatcccctactacgtgggccctctggccaggggaaac
agcagattcgcctggatgaccagaaagagcgaggaaaccatcaccccctggaacttcgaggaagtggtggacaagggcgcttccgcccagag
cttcatcgagcggatgaccaacttcgataagaacctgcccaacgagaaggtgctgcccaagcacagcctgctgtacgagtacttcaccgtgtataa
cgagctgaccaaagtgaaatacgtgaccgagggaatgagaaagcccgccttcctgagcggcgagcagaaaaaggccatcgtggacctgctgtt
caagaccaaccggaaagtgaccgtgaagcagctgaaagaggactacttcaagaaaatcgagtgcttcgactccgtggaaatctccggcgtgga
agatcggttcaacgcctccctgggcacataccacgatctgctgaaaattatcaaggacaaggacttcctggacaatgaggaaaacgaggacattc
tggaagatatcgtgctgaccctgacactgtttgaggacagagagatgatcgaggaacggctgaaaacctatgcccacctgttcgacgacaaagtg
atgaagcagctgaagcggcggagatacaccggctggggcaggctgagccggaagctgatcaacggcatccgggacaagcagtccggcaag
acaatcctggatttcctgaagtccgacggcttcgccaacagaaacttcatgcagctgatccacgacgacagcctgacctttaaagaggacatccag
aaagcccaggtgtccggccagggcgatagcctgcacgagcacattgccaatctggccggcagccccgccattaagaagggcatcctgcagaca
gtgaaggtggtggacgagctcgtgaaagtgatgggccggcacaagcccgagaacatcgtgatcgaaatggccagagagaaccagaccaccc
agaagggacagaagaacagccgcgagagaatgaagcggatcgaagagggcatcaaagagctgggcagccagatcctgaaagaacacccc
gtggaaaacacccagctgcagaacgagaagctgtacctgtactacctgcagaatggcgggatatgtacgtggaccaggaactggacatcaac
 
cggctgtccgactacgatgtggaccatatcgtgcctcagagctttctgaaggacgactccatcgacaacaaggtgctgaccagaagcgacaagaa
ccggggcaagagcgacaacgtgccctccgaagaggtcgtgaagaagatgaagaactactggcggcagctgctgaacgccaagctgattaccc
agagaaagttcgacaatctgaccaaggccgagagaggcggcctgagcgaactggataaggccggcttcatcaagagacagctggtggaaacc
cggcagatcacaaagcacgtggcacagatcctggactcccggatgaacactaagtacgacgagaatgacaagctgatccgggaagtgaaagt
gatcaccctgaagtccaagctggtgtccgatttccggaaggatttccagttttacaaagtgcgcgagatcaacaactaccaccacgcccacgacgc
ctacctgaacgccgtcgtgggaaccgccctgatcaaaaagtaccctaagctggaaagcgagttcgtgtacggcgactacaaggtgtacgacgtgc
ggaagatgatcgccaagagcgagcaggaaatcggcaaggctaccgccaagtacttcttctacagcaacatcatgaactttttcaagaccgagatt
accctggccaacggcgagatccggaagcggcctctgatcgagacaaacggcgaaaccggggagatcgtgtgggataagggccgggattttgc
caccgtgcggaaagtgctgagcatgccccaagtgaatatcgtgaaaaagaccgaggtgcagacaggcggcttcagcaaagagtctatcctgccc
aagaggaacagcgataagctgatcgccagaaagaaggactgggaccctaagaagtacggcggcttcgacagccccaccgtggcctattctgtg
ctggtggtggccaaagtggaaaagggcaagtccaagaaactgaagagtgtgaaagagctgctggggatcaccatcatggaaagaagcagcttc
gagaagaatcccatcgactttctggaagccaagggctacaaagaagtgaaaaaggacctgatcatcaagctgcctaagtactccctgttcgagct
ggaaaacggccggaagagaatgctggcctctgccggcgaactgcagaagggaaacgaactggccctgccctccaaatatgtgaacttcctgtac
ctggccagccactatgagaagctgaagggctcccccgaggataatgagcagaaacagctgtttgtggaacagcacaagcactacctggacgag
atcatcgagcagatcagcgagttctccaagagagtgatcctggccgacgctaatctggacaaagtgctgtccgcctacaacaagcaccgggataa
gcccatcagagagcaggccgagaatatcatccacctgtttaccctgaccaatctgggagcccctgccgccttcaagtactttgacaccaccatcgac
cggaagaggtacaccagcaccaaagaggtgctggacgccaccctgatccaccagagcatcaccggcctgtacgagacacggatcgacctgtct
cagctgggaggcgacaaaaggccggcggccacgaaaaaggccggccaggcaaaaaagaaaaaggtgagcgctggaggaggtggaagc
ggaggaggaggaagcggaggaggaggtagcctcgagaccatgccaagcaagaaaagcggcccgcaaccccataagaggtgggtgttcacc
cttaataatccttccgaggaggagaaaaacaaaatacgggagcttccaatctccctttttgattattttgtttgcggagaggaaggtttggaagagggta
gaactcctcacctccaggggtttgcgaattttgctaagaagcagacttttaacaaggtgaagtggtattttggtgcccgctgccacatcgagaaagcg
aaaggaaccgaccagcagaataaagaatattgcagtaaagaaggtcacatacttatcgagtgtggagcccccggaaccaggggaagcgcag
cgacctgtctactgctgtgagtacccttttggagacggggtctttggtgactgtagccgagcagttccctgtaacgtatgtgagaaatttccgggggctg
gctgaacttttgaaagtgagcgggaagatgcagcagcgtgattggaagacagctgtacacgtcatagtgggaccgcccggttgtgggaagagcc
agtgggcccgtaattttactgagcctagcgacacctactggaagcctagtagaaataagtggtgggatggctatcatggagaagaagttgttgttttgg
atgatttttatggctggttaccttgggatgatctactgagactgtgtgaccggtatccattgactgtagagactaaaggcggtactgttcctttttggcccgc
agtattttgattaccagcaatcaggcccccaggaatggtactcctcaactgctgtcccagccgtagaagctctctatcggaggattactactttgcaat
tttggaagactgctggagaacaatccacggaggtgcccgaaggccgatttgaagcagtggacccaccctgtgcccttttcccatataaaataaatta
ctga
exoI gene from bacteriophage λ (SEQ ID NO: 11) 
atgacaccggacattatcctgcagcgtaccgggatcgatgtgagagctgtcgaacagggggatgatgcgtggcacaaattacggctcggcgtcat
caccgcttcagaagttcacaacgtgatagcaaaccccgctccggaaagaagtggcctgacatgaaaatgtcctacttccacaccctgcttgctga
ggtttgcaccggtgtggctccggaagttaacgctaaagcactggcctggggaaaacagtacgagaacgacgccagaaccctgtttgaattcacttc
cggcgtgaatgttactgaatccccgatcatctatcgcgacgaaagtatgcgtaccgcctgctctcccgatggtttatgcagtgacggcaacggccttg
aactgaaatgcccgtttacctcccgggatttcatgaagttccggctcggtggtttcgaggccataaagtcagcttacatggcccaggtgcagtacagc
atgtgggtgacgcgaaaaaatgcctggtactttgccaactatgacccgcgtatgaagcgtgaaggcctgcattatgtcgtgattgagcggatgaaa
agtacatggcgagttttgacgagatcgtgccggagttcatcgaaaaaatggacgaggcactggctgaaattggttttgtatttggggagcaatggcg
atga
sgRNA comprising multiple stem-loops (SEQ ID NO: 12) 
gttttagagctaggccaacatgaggatcacccatgtctgcagggcctagcaagttaaaataaggctagtccgttatcaactttaaggagtttatatgga
aacccttaaagtggcaccgagtcggtgctaccgccgacaacgcggtttttttttt
triple fusion of E.coli recA gene (SEQ ID NO: 13) 
atggacgagaacaagaagcgcgccctggccgcggccctgggacagatcgaacgccaattcggcaaaggcgcggtcatgcgcatgggcgacc
atgagcgccaggcgatcccggccatctccaccggctccctgggtctggacatcgccctcggcatcggcggcctgcccaagggccggatcgtcga
gatctacggtccggaatcctcgggcaagaccaccctgaccctctcggtgatcgccgaggcccagaaacagggcgccacctgtgccttcgtcgacg
ccgagcacgcgctcgatcccgactatgccggcaagctgggcgtcaacgtcgacgacctgctggtctcccagccggacaccggcgagcaggccc
tggaaatcaccgacatgctggtgcgctccaacgcggtcgacgtgatcatcgtcgactccgtggccgcgctggtacccaaggccgagatcgaaggc
gagatgggcgacgcccacgtcggcctgcaggcacgcctgatgtcccaggcgctgcgcaagatcaccggcaatatcaagaacgccaactgcctg
gtcatcttcatcaaccagatccgcatgaagatcggcgtcatgttcggcaacccggaaaccaccaccggcggtaacgcactgaagttctacgcctcg
gtccgcctggacatccgtcgtaccggcgcggtgaaggaaggcgacgaggtggtgggtagcgaaacccgcgtcaaggtggtgaagaacaaggtt
tccccgccgttccgccaggccgagttccagatcctctacggtaagggcatctaccgtaccggcgagatcatcgatctgggcgtgcaattgggcctgg
tcgagaagtccggcgcctggtacagctaccagggcagcaagatcggccagggcaaggcgaacgccgccaagtacctggaagacaatccgga
aatcggttcggtactggagaagaccattcgcgaccagttgctggccaagagcggcccggtgaaggccgacgccgaagaagtggctgacgccga
agccgattcagagctcggagaaggtcaaggacagggacaaggtccaggacgaggatacgcatataagcttgacgagaacaagaagcgcgcc
ctggccgcggccctgggacagatcgaacgccaattcggcaaaggcgcggtcatgcgcatgggcgaccatgagcgccaggcgatcccggccat
ctccaccggctccctgggtctggacatcgccctcggcatcggcggcctgcccaagggccggatcgtcgagatctacggtccggaatcctcgggca
agaccaccctgaccctctcggtgatcgccgaggcccagaaacagggcgccacctgtgccttcgtcgacgccgagcacgcgctcgatcccgacta
tgccggcaagctgggcgtcaacgtcgacgacctgctggtctcccagccggacaccggcgagcaggccctggaaatcaccgacatgctggtgcg
ctccaacgcggtcgacgtgatcatcgtcgactccgtggccgcgctggtacccaaggccgagatcgaaggcgagatgggcgacgcccacgtcgg
 
cctgcaggcacgcctgatgtcccaggcgctgcgcaagatcaccggcaatatcaagaacgccaactgcctggtcatcttcatcaaccagatccgca
tgaagatcggcgtcatgttcggcaacccggaaaccaccaccggcggtaacgcactgaagttctacgcctcggtccgcctggacatccgtcgtacc
ggcgcggtgaaggaaggcgacgaggtggtgggtagcgaaacccgcgtcaaggtggtgaagaacaaggtttccccgccgttccgccaggccga
gttccagatcctctacggtaagggcatctaccgtaccggcgagatcatcgatctgggcgtgcaattgggcctggtcgagaagtccggcgcctggtac
agctaccagggcagcaagatcggccagggcaaggcgaacgccgccaagtacctggaagacaatccggaaatcggttcggtactggagaaga
ccattcgcgaccagttgctggccaagagcggcccggtgaaggccgacgccgaagaagtggctgacgccgaagccgattcaggatccggagaa
ggtcaaggacagggacaaggtccaggacgaggatacgcatatgcatgcgacgagaacaagaagcgcgccctggccgcggccctgggacag
atcgaacgccaattcggcaaaggcgcggtcatgcgcatgggcgaccatgagcgccaggcgatcccggccatctccaccggctccctgggtctgg
acatcgccctcggcatcggcggcctgcccaagggccggatcgtcgagatctacggtccggaatcctcgggcaagaccaccctgaccctctcggtg
atcgccgaggcccagaaacagggcgccacctgtgccttcgtcgacgccgagcacgcgctcgatcccgactatgccggcaagctgggcgtcaac
gtcgacgacctgctggtctcccagccggacaccggcgagcaggccctggaaatcaccgacatgctggtgcgctccaacgcggtcgacgtgatca
tcgtcgactccgtggccgcgctggtacccaaggccgagatcgaaggcgagatgggcgacgcccacgtcggcctgcaggcacgcctgatgtccc
aggcgctgcgcaagatcaccggcaatatcaagaacgccaactgcctggtcatcttcatcaaccagatccgcatgaagatcggcgtcatgttcggc
aacccggaaaccaccaccggcggtaacgcactgaagttctacgcctcggtccgcctggacatccgtcgtaccggcgcggtgaaggaaggcgac
gaggtggtgggtagcgaaacccgcgtcaaggtggtgaagaacaaggtttccccgccgttccgccaggccgagttccagatcctctacggtaaggg
catctaccgtaccggcgagatcatcgatctgggcgtgcaattgggcctggtcgagaagtccggcgcctggtacagctaccagggcagcaagatcg
gccagggcaaggcgaacgccgccaagtacctggaagacaatccggaaatcggttcggtactggagaagaccattcgcgaccagttgctggcca
agagcggcccggtgaaggccgacgccgaagaagtggctgacgccgaagccgattaa
triple fusion of human rad51 gene (SEQ ID NO: 14) 
atggcaatgcagatgcagcttgaagcaaatgcagatacttcagtggaagaagaaagctttggcccacaacccatttcacggttagagcagtgtggc
ataaatgccaacgatgtgaagaaattggaagaagctggattccatactgtggaggctgttgcctatgcgccaaagaaggagctaataaatattaag
ggaattagtgaagccaaagctgataaaattctggctgaggcagctaaattagttccaatgggtttcaccactgcaactgaattccaccaaaggcggt
cagagatcatacagattactactggctccaaagagcttgacaaactacttcaaggtggaattgagactggatctatcacagaaatgtttggagaattc
cgaactgggaagacccagatctgtcatacgctagctgtcacctgccagcttcccattgaccggggtggaggtgaaggaaaggccatgtacattgac
actgagggtacctttaggccagaacggctgctggcagtggctgagaggtatggtctctctggcagtgatgtcctggataatgtagcatatgctcgagc
gttcaacacagaccaccagacccagctcctttatcaagcatcagccatgatggtagaatctaggtatgcactgcttattgtagacagtgccaccgccc
tttacagaacagactactcgggtcgaggtgagctttcagccaggcagatgcacttggccaggtttctgcggatgcttctgcgactcgctgatgagtttg
gtgtagcagtggtaatcactaatcaggtggtagctcaagtggatggagcagcgatgtttgctgctgatcccaaaaaacctattggaggaaatatcatc
gcccatgcatcaacaaccagattgtatctgaggaaaggaagaggggaaaccagaatctgcaaaatctacgactctccctgtcttcctgaagctga
agctatgttcgccattaatgcagatggagtgggagatgccaaagacgagctcggagaaggtcaaggacagggacaaggtccaggacgaggat
acgcatataagcttatggcaatgcagatgcagcttgaagcaaatgcagatacttcagtggaagaagaaagctttggcccacaacccatttcacggtt
agagcagtgtggcataaatgccaacgatgtgaagaaattggaagaagctggattccatactgtggaggctgttgcctatgcgccaaagaaggagc
taataaatattaagggaattagtgaagccaaagctgataaaattctggctgaggcagctaaattagttccaatgggtttcaccactgcaactgaattcc
accaaaggcggtcagagatcatacagattactactggctccaaagagcttgacaaactacttcaaggtggaattgagactggatctatcacagaaa
tgtttggagaattccgaactgggaagacccagatctgtcatacgctagctgtcacctgccagcttcccattgaccggggtggaggtgaaggaaagg
ccatgtacattgacactgagggtacctttaggccagaacggctgctggcagtggctgagaggtatggtctctctggcagtgatgtcctggataatgtag
catatgctcgagcgttcaacacagaccaccagacccagctcctttatcaagcatcagccatgatggtagaatctaggtatgcactgcttattgtagac
agtgccaccgccctttacagaacagactactcgggtcgaggtgagctttcagccaggcagatgcacttggccaggtttctgcggatgcttctgcgact
cgctgatgagtttggtgtagcagtggtaatcactaatcaggtggtagctcaagtggatggagcagcgatgtttgctgctgatcccaaaaaacctattgg
aggaaatatcatcgcccatgcatcaacaaccagattgtatctgaggaaaggaagaggggaaaccagaatctgcaaaatctacgactctccctgtc
ttcctgaagctgaagctatgttcgccattaatgcagatggagtgggagatgccaaagacggatccggagaaggtcaaggacagggacaaggtcc
aggacgaggatacgcatatgcatgcatggcaatgcagatgcagcttgaagcaaatgcagatacttcagtggaagaagaaagctttggcccacaa
cccatttcacggttagagcagtgtggcataaatgccaacgatgtgaagaaattggaagaagctggattccatactgtggaggctgttgcctatgcgcc
aaagaaggagctaataaatattaagggaattagtgaagccaaagctgataaaattctggctgaggcagctaaattagttccaatgggtttcaccact
gcaactgaattccaccaaaggcggtcagagatcatacagattactactggctccaaagagcttgacaaactacttcaaggtggaattgagactgga
tctatcacagaaatgtttggagaattccgaactgggaagacccagatctgtcatacgctagctgtcacctgccagcttcccattgaccggggtggagg
tgaaggaaaggccatgtacattgacactgagggtacctttaggccagaacggctgctggcagtggctgagaggtatggtctctctggcagtgatgtc
ctggataatgtagcatatgctcgagcgttcaacacagaccaccagacccagctcctttatcaagcatcagccatgatggtagaatctaggtatgcact
gcttattgtagacagtgccaccgccctttacagaacagactactcgggtcgaggtgagctttcagccaggcagatgcacttggccaggtttctgcgga
tgcttctgcgactcgctgatgagtttggtgtagcagtggtaatcactaatcaggtggtagctcaagtggatggagcagcgatgtttgctgctgatcccaa
aaaacctattggaggaaatatcatcgcccatgcatcaacaaccagattgtatctgaggaaaggaagaggggaaaccagaatctgcaaaatctac
gactctccctgtcttcctgaagctgaagctatgttcgccattaatgcagatggagtgggagatgccaaagactga
MS2-derived stem-loop for binding (SEQ ID NO: 15)
   ggccaacatgaggatcacccatgtctgcagggcc
PP7-derived stem-loop for binding (SEQ ID NO: 16)
   taaggagtttatatggaaaccctta
P22-derived stem-loop for binding bacteriophage B-box (SEQ ID NO: 17)
   accgccgacaacgcggt
MS2 coat protein (SEQ ID NO: 18) 
atggcttcaaactttactcagttcgtgctcgtggacaatggtgggacaggggatgtgacagtggctccttctaatttcgctaatggggtggcagagtgg
atcagctccaactcacggagccaggcctacaaggtgacatgcagcgtcaggcagtctagtgcccagaagagaaagtataccatcaaggtggag
gtccccaaagtggctacccagacagtgggcggagtcgaactgcctgtcgccgcttggagatcctacctgaacatggaactcactatcccaattttcg
ctaccaattctgactgtgaactcatcgtgaaggcaatgcaggggctcctcaaagacggcaatcctatcccttccgccatcgccgctaactcaggca
PP7 bacteriophage coat protein (SEQ ID NO: 19) 
atgtccaaaaccatcgttctttcggtcggcgaggctactcgcactctgactgagatccagtccaccgcagaccgtcagatcttcgaagagaaggtcg
ggcctctggtgggtcggctgcgcctcacggcttcgctccgtcaaaacggagccaagaccgcgtatcgagtcaacctaaaactggatcaggcggac
gtcgttgattgctccaccagcgtctgcggcgagcttccgaaagtgcgctacactcaggtatggtcgcacgacgtgacaatcgttgcgaatagcaccg
aggcctcgcgcaaatcgttgtacgatttgaccaagtccctcgtcgcgacctcgcaggtcgaagatcttgtcgtcaaccttgtgccgctgggccgttaa
P22 bacteriophage coat protein (SEQ ID NO: 20) 
atgacggttatcacctacgggaagtcaacgtttgcaggcaatgctaaaactcgccgtcatgagcggcgcagaaagctagccatagagcgcgaca
ccatctgcaatatcatcgattcaatttttggctgcgatgctcctgatgcttctcaggaagttaaagccaaaagaattgaccgtgtcaccaaagccatttcg
cttgccggaacgcgtcagaaggaagttgaaggaggatctgtacttcttccaggcgtagcactttacgcggctggtcatcgtaagagcaaacaaata
acagcgaggtaa
MSH2 dominant-negative gene sequence (M688R) (SEQ ID NO: 21)

atggcggtgcagccgaaggagacgctgcagttggagagcgcggccgaggtcggcttcgtgcgcttctttcagggcatgccggagaagccgacca
ccacagtgcgccttttcgaccggggcgacttctatacggcgcacggcgaggacgcgctgctggccgcccgggaggtgttcaagacccaggggggt
gatcaagtacatggggccggcaggagcaaagaatctgcagagtgttgtgcttagtaaaatgaattttgaatcttttgtaaaagatcttcttctggttcgtc
agtatagagttgaagtttataagaatagagctggaaataaggcatccaaggagaatgattggtatttggcatataaggcttctcctggcaatctctctc
agtttgaagacattctctttggtaacaatgatatgtcagcttccattggtgttgtgggtgttaaaatgtccgcagttgatggccagagacaggttggagttg
ggtatgtggattccatacagaggaaactaggactgtgtgaattccctgataatgatcagttctccaatcttgaggctctcctcatccagattggaccaaa
ggaatgtgttttacccggaggagagactgctggagacatggggaaactgagacagataattcaaagaggaggaattctgatcacagaaagaaa
aaaagctgacttttccacaaaagacatttatcaggacctcaaccggttgttgaaaggcaaaaagggagagcagatgaatagtgctgtattgccaga
aatggagaatcaggttgcagtttcatcactgtctgcggtaatcaagtttttagaactcttatcagatgattccaactttggacagtttgaactgactactttg
acttcagccagtatatgaaattggatattgcagcagtcagagcccttaaccttttcagggttctgttgaagataccactggctctcagtctctggctgcctt
gctgaataagtgtaaaacccctcaaggacaaagacttgttaaccagtggattaagcagcctctcatggataagaacagaatagaggagagattga
atttagtggaagcttttgtagaagatgcagaattgaggcagactttacaagaagatttacttcgtcgattcccagatcttaaccgacttgccaagaagttt
caaagacaagcagcaaacttacaagattgttaccgactctatcagggtataaatcaactacctaatgttatacaggctctggaaaaacatgaagga
aaacaccagaaattattgttggcagtttttgtgactcctcttactgatcttcgttctgacttctccaagtttcaggaaatgatagaaacaactttagatatgg
atcaggtggaaaaccatgaattccttgtaaaaccttcatttgatcctaatctcagtgaattaagagaaataatgaatgacttggaaaagaagatgcag
tcaacattaataagtgcagccagagatcttggcttggaccctggcaaacagattaaactggattccagtgcacagtttggatattactttcgtgtaacct
gtaaggaagaaaaagtccttcgtaacaataaaaactttagtactgtagatatccagaagaatggtgttaaatttaccaacagcaaattgacttctttaa
atgaagagtataccaaaaataaaacagaatatgaagaagcccaggatgccattgttaaagaaattgtcaatatttcttcaggctatgtagaaccaat
gcagacactcaatgatgtgttagctcagctagatgctgttgtcagctttgctcacgtgtcaaatggagcacctgttccatatgtacgaccagccattttgg
agaaaggacaaggaagaattatattaaaagcatccaggcatgcttgtgttgaagttcaagatgaaattgcatttattcctaatgacgtatactttgaaa
aagataaacagatgttccacatcattactggccccaatatgggaggtaaatcaacatatattcgacaaactggggtgatagtactccgggcccaaat
tgggtgttttgtgccatgtgagtcagcagaagtgtccattgtggactgcatcttagcccgagtaggggctggtgacagtcaattgaaaggagtctccac
gttcatggctgaaatgttggaaactgcttctatcctcaggtctgcaaccaaagattcattaataatcatagatgaattgggaagaggaacttctacctac
gatggatttgggttagcatgggctatatcagaatacattgcaacaaagattggtgctttttgcatgtttgcaacccattttcatgaacttactgccttggcca
atcagataccaactgttaataatctacatgtcacagcactcaccactgaagagaccttaactatgctttatcaggtgaagaaaggtgtctgtgatcaaa
gttttgggattcatgttgcagagcttgctaatttccctaagcatgtaatagagtgtgctaaacagaaagccctggaacttgaggagtttcagtatattgga
gaatcgcaaggatatgatatcatggaaccagcagcaaagaagtgctatctggaaagagagaacctcagagtgacagagccaaaagaccagtg
cctcatttgctgacatggaaaaggaaacttcgtgggggaaagagatctgcttgcagtcggccagagagacagaaccagggcagtgcgactcca
agtgcatcagcatag

## Claims

1. A nucleic acid encoding a first fusion protein comprising a 5' to 3' DNA exonuclease domain and an RNA binding domain.

2. A nucleic acid according to claim 2, wherein the exonuclease is Exo1.

3. A nucleic acid according to claim 1 or 2, for inhibition of NHEJ during transformation of a cellular genome so as to promote HDR.

4. A nucleic acid according to any of claims 1 to 3, for transformation of a eukaryotic cell in concert with an RNA-guided endonuclease.

5. A nucleic acid encoding a second fusion protein comprising an endonuclease domain fused to a binding domain for an origin of replication.

6. A nucleic acid encoding a third fusion protein comprising a recombination inducing domain and an RNA binding domain.

7. A nucleic acid encoding a fourth fusion protein comprising a domain comprising an inhibitor of the mismatch repair pathway; and an RNA binding domain.

8. A nucleic acid according to claims 1-4, 6 or 7, wherein the RNA binding domain binds to a tracrRNA for use in CRISPR-Cas transformation.

9. A method of transforming the genome of a human or animal cell comprising the steps of:
a. expressing an RNA-guided endonuclease in the cell;
b. expressing in the cell or introducing into the cell a sequence specific guide RNA to direct cleavage by the endonuclease domain to a specific locus
c. expressing in the cell the nucleic acid of any of claims 1 to 4 or introducing into the cell the first fusion protein of any of claims 1 to 4.

10. A method according to claim 9, wherein the exonuclease is a dsDNA exonuclease.

11. A method according to claim 9 or 10 for modifying a genome, wherein a double strand break is introduced into the genome in the presence of an exogenous donor nucleic acid sequence comprising a donor nucleic acid sequence as a template for modifying the chromosome or as an exogenous sequence to be integrated into the chromosome, and a DNA repair mechanism modifies the genome via homology-directed repair (HDR), the method comprising:
suppressing non-homologous end joining (NHEJ) repair of the break to promote repair by HDR by expressing in the cell the nucleic acid of any of claims 1 to 4 or introducing into the cell the first fusion protein of any of claims 1 to 4.

12. A method according to claim 9, 10 or 11, further comprising the steps of:
a. expressing in the cell one or more nucleic acids of claims 5 to 8; or
b. introducing into the cell one or more fusion proteins of claims 5 to 8.

13. A method according to claim 12, comprising expressing in the cell two or more nucleic acids of claims 1 to 4 and 6 to 8 or introducing into the cell two or more fusion proteins of claims 1 to 4 and 6 to 8, wherein the RNA binding protein domains of the respective fusion proteins bind to different RNA sequences.

14. A fusion protein comprising a 5' to 3' DNA exonuclease domain and an RNA binding domain.

15. Use of the fusion protein of claim 14, or a nucleic acid encoding the fusion protein in transformation of a human or animal cell using an RNA-guided endonuclease.
